# EUROPEAN PATENT SPECIFICATION

(11) **EP 0 756 010 B1**
(45) Date of publication and mention of the grant of the patent: **04.07.2001**
(21) Application number: 96114821.0
(22) Date of filing: 09.01.1984
(51) Int. Cl.: C12Q 1/68

(54) **Method for detecting, identifying, and quantitating organisms and viruses**
Verfahren zum Nachweis, Identifizieren und Quantifizieren von Organismen und Viren
Procédé de détection, identification et quantification d'organismes et de virus

(30) Priority: 10.01.1983 US 456729
(43) Date of publication of application: 29.01.1997
(62) Divisional of application: 92114515.7
(73) Proprietor: GEN-PROBE INCORPORATED, San Diego, California 92121 (US)
(72) Inventor: Kohne, David E.E., La Jolla, CA 92037 (US)
(74) Representative: Maschio, Antonio

(56) References cited:
- EP-A- 0 155 359
- EP-A- 0 155 360
- WIRTH D ET AL: "Rapid identification of Leishmania species by specific hybridization of kinetoplast DNa in cutaneous lesions" PROC. NATL. ACAD. SCI. USA, vol. 79, November 1982, pages 6999-7003, XP002082053
- AMIKAM D ET AL: "Ribosomal RNA genes in mycoplasma" NUCLEIC ACIDS RESEARCH, vol. 10, no. 14, 24 July 1982, pages 4215-22, XP002082054
- BRENNER D ET AL: "Conservation of transfer ribonucleic acid and 5S ribonucleic acid cistrons in Enterobacteriaceae" J. BACTERIOLOGY, vol. 129, no. 3, March 1977, pages 1435-39, XP002082055
- GARVIE E ET AL: "Sub-divisions within the Genus Streptococcus using deoxyribonucleic acid/ribosomal ribonucleic acid hybridization" ZENRTALBLATT FÜR BAKTERIOLOGIE MIKROBIOLOGIE UND HYGIENE, I. ABT. ORIG. C, vol. 2, no. 4, 1981, pages 299-374, XP002082056

## Description

### TECHNICAL FIELD

The invention relates to a method and means for detecting, identifying, and quantitating organisms in biological and other samples. Thus, it relates to a method for specifically and sensitively detecting and quantitating any organism containing ribosomal RNA, (hereinafter R-RNA), any members of large, intermediate, or small sized categories or taxonomic groups of such organisms; and previously unknown organisms containing R-RNA. The method is capable of detecting the presence of even one organism, containing R-RNA.

My invention and the novelty, utility, and unobviousness thereof can be more clearly understood and appreciated when considered in light of the representative background information hereinafter set out, comprising this art.

### BACKGROUND ART

Each of the cells of all life forms, except viruses, contain ribosomes and therefore ribosomal RNA. A ribosome contains three separate single strand RNA molecules, namely, a large molecule, a medium sized molecule, and a small molecule. The two larger R-RNA molecules vary in size in different organisms.

Ribosomal RNA is a direct gene product and is coded for by the R-RNA gene. This DNA sequence is used as a template to synthesize R-RNA molecules. A separate gene exists for each of the ribosomal RNA subunits. Multiple R-RNA genes exist in most organisms, many higher organisms containing both nuclear and mitochondrial R-RNA genes. Plants and certain other forms contain nuclear, mitochondrial and chloroplast R-RNA genes. For simplicity of discussion hereinafter, the three separate R-RNA genes will be referred to as the R-RNA gene.

Numerous ribosomes are present in all cells of all life forms. About 85-90 percent of the total RNA in a typical cell is R-RNA. A bacterium such as E. coli contains about 10⁴ ribosomes per cell While a mammalian liver cell contains about 5 x 10⁶ ribosomes. Since each ribosome contains one of each R-RNA subunit, the bacterial cell and mammalian cell contain 10⁴ and 5 x 10⁶, respectively, of each R-RNA subunit.

Nucleic acid hybridization, a procedure well-known in the art, has been used in the prior art to specifically detect extremely small or large quantities of a particular nucleic acid sequence, even in the presence of a very large excess of non-related sequences. Prior art uses of nucleic acid hybridization are found, for example, in publications involving molecular genetics of cells and viruses, genetic expression of cells and viruses; genetic analysis of life forms; evolution and taxonomy or organisms and nucleic acid sequences; molecular mechanisms of disease processes; diagnostic methods for specific purposes, including the detection of viruses and bacteria in cells and organisms.

Probably the best characterized and most studied gene and gene product are the R-RNA gene and R-RNA, and the prior art includes use of hybridization of R-RNA and ribosomal genes in genetic analysis and evolution and taxonomic classification of organisms and ribosomal gene sequences. Genetic analysis includes, for example, the determination of the numbers of ribosomal RNA genes in various organisms; the determination of the similarity between the multiple ribosomal RNA genes which are present in cells; determination of the rate and extent of synthesis of R-RNA in cells and the factors which control them. Evolution and taxonomic studies involve comparing the R-RNA gene base sequence from related and widely different organisms.

It is known that the ribosomal RNA gene base sequence is at least partially similar in widely different organisms, and that the DNA of E. coli bacterial ribosomal RNA genes hybridizes well with R-RNA from plants, mammals, and a wide variety of other bacterial species. The fraction of the E. coli gene which hybridizes to these other species varies with the degree of relatedness of the organisms. Virtually all of the R-RNA gene sequence hybridizes to R-RNA from closely related bacterial species, while less hybridizes to R-RNA from distantly related bacterial species, and even less with mammalian R-RNA.

The sensitivity and ease of detection of members of specific groups of organisms by utilizing probes specific for the R-RNA of that group of organisms is greatly enhanced by the large number of R-RNA molecules which are present in each cell. In addition the hybridization test is made significantly easier since RNA molecules present in cells are single stranded. Thus a denaturation step, such as must be used for a hybridization test which detects any fraction of cell DNA, is not necessary when the target molecule is RNA.

My invention also relates therefore, to a method for specifically and sensitively detecting, identifying and quantitating organisms present in cells. More particularly, the method is useful for sensitively detecting, identifying and quantitating any member of different sized categories of organisms, eukaryotic cells, and in some cases previously unknown organisms containing excess RNA molecules present in R-RNA.

This invention therefore has broad application to any area in which it is important to determine the presence or absence of living organisms present in cells; the'state of genetic expression of an organism, cell, or groups of cells of prokaryotic or eukaroytic organisms. Such areas include medical, veterinary, and agricultural diagnostics and industrial and pharmaceutical quality control.

The invention involves a method for using specifically produced nucleic acids complementary to R-RNA to detect, identify and quantitate specific organisms, groups of organisms, or groups of eukaryotic cells by the process of nucleic acid hybridization.

### Prior Art Hybridization Procedures

Two basic nucleic acid hybridization procedures are disclosed in the prior art. In one, in solution hybridization, both the probe and sample nucleic acid molecules are free in solution. With the other method the sample is immobilized on a solid support and the probe is free in solution. Both of these methods are widely used and well documented in the literature. An example of the in solution method is presented hereinafter in the examples. Also, in the article by Thomas et al., Proc. Natl. Acad. Sci. USA (1980), 77, p. 520, is an example of the immobilized method.

The basic components of a nucleic acid hybridization test are:
- 1. Probe -: A marked single strand nucleic acid sequence which is complementary to the nucleic acid sequences to be detected (that is the target sequences). As used herein, the target sequence is a sub-sequence of R-RNA.

The probe length can vary from 5 bases to tens of thousands of bases, and will depend upon the specific test to be done. Only part of the probe molecule need be complementary to the nucleic acid sequence to be detected (hereinafter the target sequences). In addition, the complementarity between the probe and the target sequence need not be perfect. Hybridization does occur between imperfectly complementary molecules with the result that a certain fraction of the bases in the hybridized region are not paired with the proper complementary base. A probe may.be composed of either RNA or DNA. The form of the nucleic acid probe may be a marked single strand molecule of just one polarity or marked single strand molecule having both polarities present. The form of the probe, like its length, will be determined..by the type of hybridization test to be done.
- 2. Sample -: The sample may or may not contain the target molecule (i.e. the organism of interest). The sample may take a variety of forms, including liquid such as water or serum, or solid such as dust, soil or tissue samples. The s'ample nucleic acid must be made available to contact the probe before any hybridization of probe and target molecule can occur. Thus the organism's RNA must be free from the cell and placed under the proper conditions before hybridization can occur. Prior art methods of in solution hybridization necessitate the purification of the RNA in order to be able to obtain hybridization of the sample R-RNA with the probe. This has meant that to utilize the in solution method for detecting target sequences in a sample, the nucleic acids of the sample must first be purified to eliminate protein, lipids, and other cell components, and then contacted with the probe under hybridization conditions. The purifications of the sample nucleic acid takes at least several hours and can take up to a day, depending on the nature and quantity of the sample.
- 3. Hybridization Method -: Probe and sample must be mixed under conditions which will permit nucleic acid hybridization. This involves contacting the probe and sample in the presence of an inorganic or organic salt under the proper concentration and temperature conditions. The probe and sample nucleic acids must be in contact for a long enough time that any possible hybridization between the probe and sample nucleic acid may occur.

The concentration of probe or target in the mixture will determine the time necessary for hybridization to occur. The higher the probe or target concentration the shorter the hybridization incubation time needed.

A nucleic acid hybridization incubtation mixture composed of probe and sample nucleic acids must be incubated at a specific temperature for a long enough time for hybridization to occur. The length of time necessary for hybridization to complete depends upon the concentration of the probe nucleic acid, the concentration of the sample nucleic acid which is complementary to the probe, and a basic rate of hybridization which is characteristic of the hybridization conditions used. The basic rate of hybridization is determined by the type of salt present in the incubation mix, its concentration, and the temperature of incubation. Sodium chloride, sodium phosphate and sodium citrate are the salts most frequently used for hybridization and the salt concentration used is rarely above 1 M and sometimes as high as 1.5 - 2 M. The salts mentioned above yield comparable rates of nucleic acid hybridization when used at the same concentrations and temperatures, as do the comparable potassium, lithium, rubidium, and cesium salts. Britten et al. (1974) (Methods in Enzymology, Volume XXIX, part E., ed. Grossman and Moldave; Academic Press, New York, page 364) and Wetmur and Davidson (1968) (J. Molecular Biology, Vol. 31, page 349) present data which illustrates the standard basic rates of hybridization attained in commonly used salts. The hybridization rates of DNA with RNA vary somewhat from those of DNA hybridizing with DNA. The magnitude of the variation is rarely over tenfold and varies, depending for example, on whether an excess of DNA or RNA is used. See Galau et al. (1977) (Proc. Natl. Acad. Sci. USA,. Vol 74, #6, pg. 2306).

Certain conditions result in the acceleration of DNA:DNA hybridization. An emulsion of phenol and salt promotes the very rapid hybridization of DNA when the mixture is agitated. Rate increases several thousand times faster than standard DNA hybridization rates are attained with this system (Kohne et al., Biochemistry (1977) Vol. 16, p. 532a). DNA hybridization rate acceleration of 50 to 100 fold over the standard rates has also been observed when neutral and anionic dextran polymers were mixed with single strand DNA in solution (Wetmur, Biopolymers (1975) Vol 14. p. 2517). Neither of these DNA accelerated rate condition was reported to accelerate the hybridization rate of DNA:RNA hybridization. I am not aware of any prior art which documents a condition for accelerating the rate of RNA:DNA hybridization.
- 4. Hybridization Assay -: A procedure is needed to detect the presence of probe molecules hybridized to the target molecules. Such a method depends upon the ability to separate probe which is hybridized to target molecules from probe which is not hybridized to target molecules. Prior art procedures for assaying in solution hybridization mixtures have been done on sample nucleic acids which are first purified and then contacted with the probe in the hybridization incubation mixture.

Hydroxyapatite (HA) has been used as a standard method for assaying in solution hybridization mixtures for the presence of hybridized probe. Under the proper conditions HA selectively binds hybridized DNA probe but does not bind probe which is not hybridized. Other methods are available to assay for hybridized probe. These include an S₁ nuclease assay which depends on the ability of a specific enzyme to degrade non-hybridized probe to small subunits while the hybridized probe is not degraded by the enzyme and remains large. The degraded probe can then be separated from the hybridized probe by a size separation technique. Various methods for assaying for in solution hybridized nucleic acids are presented in Britten et al. (1974) supra.

The immobilized sample nucleic acid hybridization methods have the hybridization assay built into the hybridization method. These methods involve fixing the sample nucleic acid onto an inert support and then hybridizing this immobilized nucleic acid with a marked probe which is free in solution. Hybridization of any probe with the immobilized sample nucleic acid results in the binding of the probe to the sample nucleic acid and therefore the attachment of'the probe to the inert support. Non-hybridized probe remains free in solution and can be washed away from the inert support and the hybridized probe. Such a method requires at least several hours to prepare the sample for nucleic acid hybridization and_one to two hours of washing and utilizes large amounts of probe. An advantage of this method is the capability to place multiple samples on the same inert support and to hybridize and process all the. samples at one time. Examples of such an immobilized sample method is presented in Analytical Biochemistry (1983) Vol. 128, p. 415, and J. of Infectious Disease (1982) Vol. 145, #6, p. 863.

### Making Nucleic Acids Available for Hybridization

In solution nucleic acid hybridization methods have always utilized nucleic acids which have been purified away from other cell components. Nucleic acids in cells and viruses are normally tightly complexed with other cell components, usually protein, and, in this form are not available for hybridization. Simply breaking the cell or virus open to release the contents does not render the nucleic acids available for hybridization. The nucleic acids remain complexed to other cell or viral components even though released from the cell, and may in fact become extensively degraded by nucleases which also may be released. In addition a marked probe added to such a mix may become complexed to "sticky" cell or viral components and be rendered unavailable for hybridization, or the probe may be degraded by nuclease action.

A variety of prior art methods exist for purifiying nucleic acids and several of these are described in Maniatis et al., supra. These methods are all time consuming --one taking an hour is regarded as very rapid-- and require multiple manipulations.

Insofar as I am aware, there is no prior art method for performing in solution nucleic acid hybridization which does not require the use of some sort of pre-purification step to make the nucleic acids available for hybridization.

The immobilized nucleic acid hybridization methods involve fixing the sample nucleic acid onto an inert support and then hybridizing this immobilized nucleic acid with marked probe which is free in solution. The process of fixing the nucleic acids on the inert support provides a purification step effective enough. to make the bound nucleic acids available for hybridization. Most of the non-nucleic acid cell or viral components do not bind to the inert support, and those which do bind do so at a different location than the nucleic acids. Such a method requires several hours, at a minimum, to prepare the sample nucleic acids for hybridization. An advantage of this method is the ability to place multiple samples on the inert support and process them all together through the hybridization and the hybridization assay steps. The hybridization assay consists of removing the inert support from the hybridization mixture. Probe which is hybridized to the fixed sample remains with the inert support while non-hybridized probe remains free in solution.

Thus, while the presence of organisms can be detected by any one of'a large variety of prior art methods, none of these is entirely satisfactory for one reason or another. Such methods include, e.g., growth methods, optical detection methods, serologic and immunochemical methods, and biochemical methods, as shown below:

### Growth Tests:

A large number of different growth tests exist, each useful for the growth of a specific organism or group of organisms. Growth tests have the potential sensitivity to detect one organism. In practice, however, many organisms are difficult or impossible to grow. These tests are usually lengthy, taking from one day, to months, to complete. In addition, a very large number of tests would be needed to detect the presence of any member of a large group of organisms (e.g., all bacteria), assuming that the growth conditions for all members of the group are known.

### Optical Detection Methods:

Microscopic analysis coupled with differential staining methods is a very powerful, and in many cases, very rapid detection method. A major problem with this approach is the detection of specific organisms in the presence of large quantities of other organisms, for example, the identification of a specific type of gram negative rod shaped bacteria, in the presence of many different kinds of gram negative rod shaped bacteria. In addition, a large number of tests would be needed to detect the presence of all members of a large group of organisms (such as the group of all bacteria).

### Serologic and Immunochemical Methods and Biochemical Tests:

A large number of different types of these tests exist. They are usually qualitative, not very sensitive and often require a growth step. A great many of these tests would be required to detect all members of a large group of organisms.

U.S. Patent 4,358,535 to Falkow et al. discloses a method for the detection of genetic material, i.e., genes or genomes. In this patent a clinical sample or isolate suspected of containing a pathogen is transferred onto an inert porous support, such as a nitrocellulose filter, and treated in such a way that the cells are localized. The cells, are then treated in such a way as to release their DNA and cause it to couple onto the support. Subsequent treatment causes a separation of the individual DNA strands of the genome. The strands are then contacted with labelled probes specific for the characteristic polynucleotide sequence under hybridization conditions. Hybridization of the probe to the single stranded polynucleotides from the pathogen is detected by means of the label.

The method of this patent, for detecting genes or genomes, like the other methods mentioned above does not have the specificity, sensitivity, rapidity or ease of performance of that of my invention. A summary of comparisons of the Falkow et al. method as disclosed in the patent and that of applicant's method, as herein disclosed, is set out below:

| 1. | Method of doing hybridization | |
|---|---|---|
| | FALKOW ET AL. METHOD | APPLICANT'S METHOD |
| | Immobilized method only | In Solution method emphasized. Immobilized method can be used. |

| 2. | Class of nucleic acid to be detected | |
|---|---|---|
| | FALKOW ET AL. METHOD | APPLICANT'S METHOD |
| | Genetic material (i.e., genes or genomes). In cellular organisms the genetic material is always DNA. | Detection of a primary gene product (RNA) only. RNA is not present as genetic material in cellular organisms. |

| 3. | Abundance (copies per cell) of nucleic acid sequences to be detected | |
|---|---|---|
| | FALKOW ET AL. METHOD | APPLICANT'S METHOD |
| | Virtually all microorganism chromosomal genes are present only one time per cell. Extrachromosomal genes are ususally present 1-3 times per cell. Ribosomal RNA genes are present 3-6 times per cell. | 10⁴ copies of R-RNA are present per bacterial cell. The numbers are generally higher in eukaryotic cells. |

| 4. | Ability of hybridization method to quantitate nucleic acids | |
|---|---|---|
| | FALKOW ET AL. METHOD | APPLICANT'S METHOD |
| | None disclosed | Excellent ability to quantitate nucleic acids, both DNA and RNA. |

| 5. | Ability to determine and quantitate the state of genetic expression of a cell | |
|---|---|---|
| | FALKOW ET AL. METHOD | APPLICANT'S METHOD |
| | Genetic expression cannot be determined by detecting genetic material. | Genetic expression can be determined and quantitated by using probes which detect RNAs. |

| 6. | Relative probability of detecting a false positive during diagnosis | |
|---|---|---|
| | FALKOW ET AL. METHOD | APPLICANT'S METHOD |
| | High (detects only specific genes). | Low (when emphasis is on detecting RNA). |

| 7. | Relative sensitivity of detection of nucleic acids | |
|---|---|---|
| | FALKOW ET AL. METHOD | APPLICANT'S METHOD |
| | Good. Nucleic acid hybridization test are in general quite sensitive. | Highly sensitive. From 20 to 10⁴ times more sensitive than possible with the approach outlined in Falkow. RNA is almost always more abundant than the genes which make it. The in solution method also confers extra sensitivity over the immobilized method. |

| 8. | Preparation of sample for hybridization test | |
|---|---|---|
| | FALKOW ET AL. METHOD | APPLICANT'S METHOD |
| | Takes from 2 - 10 hours to immobilize sample nucleic acids and make them available for hybridization. Includes a step for converting DNA to single strand form. Not all the sample nucleic acids are capable of hybridization. | Takes 1 - 5 minutes to make sample available for hybridization. RNA in cells is already single stranded. All of the sample nucleic acid is capable of hybridizing. |
| | Amount of probe needed | |

| 9 | FALKOW ET AL. METHOD | APPLICANT'S METHOD |
|---|---|---|
| | Usually takes 0.01 to 1 micrograms of probe in hybridization mixture. | Need 10⁻⁵ to 10⁻⁶ micrograms of probe per sample. |

| 10. | Time needed for hybridization to occur | |
|---|---|---|
| | FALKOW ET AL. METHOD | APPLICANT'S METHOD |
| | 2 - 20 hours | 0.2 - 0.6 hours |

I am not aware of any prior art which teaches my method of detecting the presence or absence of R-RNA, characteristic of a particular group of organisms utilizing nucleic acid hybridization wherein is used a selected marked nucleic acid molecule complementary to a subsequence of R-RNA from a particular source. Nor am I aware of any prior art which discloses my method for detecting the presence or absence of R-RNA in general by nucleic acid hybridization using a marked nucleic acid molecule complementary to R-RNA subsequences from a specific source.

Nor am I aware of any prior art which teaches my method of detecting the presence or absence of a nucleic acid characteristic of a particular group of organisms ; or of rapidly making available for in solution nucleic acid hybridization with a specific marked probe, the nucleic acids of a particular group of organisms for any purpose; or of utilizing an in solution nucleic acid hybridization method which combines a rapid method for making the nucleic acids of specific groups of organisms available for hybridization with a specific complementary probe, with a method for detecting an organism's nucleic acid by greatly accellerating the rate of in solution hybridization of the nucleic acids of an organim and the marked probe complementary to the organism's nucleic acid; or of determining the antimicrobial agent sensitivity of a particular group of organisms ; or of assaying for the presence of antimicrobial substances in blood, urine, other body fluids or tissues or other samples; or for determining the state of growth of cells; or of detecting microorganism infections; or of rapidly assaying for the presence, in a hybridization mixture, of probe which has hybridized, by contacting the mixture with hydroxyapatite under'predetermined conditions and then processing the resulting solution in a specific manner.

### DISCLOSURE OF THE INVENTION

The present invention provides a method and means for detecting, identifying, and quantitating organisms in biological and other samples, and more particularly to a method for specifically and sensitively detecting and quantitating any organism containing the ribosomal RNA, (hereinafter R-RNA) ; any members of large, intermediate, or small sized categories or taxonomic groups of such organisms; and previously unknown organisms containing R-RNA. The method is capable of detecting the presence of even one organism, containing R-RNA.

The invention also provides a method and means having as characterizing qualities: (a) the ability to specifically detect the presence of any one of a large number of different organisms with a single assay procedure which also works regardless of the pattern of genetic expression of any particular organism; (b) the ability to modify the test to detect only specific categories of organisms, even in the presence of organisms not in the group of interest; (c) extremely high sensitivity of detection, and ability to detect the presence of one organism or cell; (d) the ability to quantitate the number of organisms or cells present; and (e) does not require a growth step.

My invention provides means for detecting the antimicrobial agent sensitivity of a particular group of organisms ; for assaying the presence of antimicrobial substances in blood, urine, other body fluids or tissues or other samples; for determining the state of growth of cells; for detecting microorganism infections; and for rapidly assaying for the presence in a hybridization mixture of probe which has hybridized.

As described hereinbefore, R-RNA base sequences are partially similar in widely different organisms. The more closely related two organisms are, the larger the fraction of the total R-RNA which is similar in the two species. The R-RNA sequence of any particular species or organism can be regarded as a series of short R-RNA subsequences, of which one subsequence is similar in virtually all life forms. Therefore, the R-RNA of almost all life forms must contain this subsequence. A different subsequence is similar only in the R-RNA of the members of the species to which that organism belongs. Other subsequences are present in the order or organisms that the species belongs to, and so on.

Because the R-RNA sequences of widely different organisms are at least partially similar, the method of my invention, using a probe which detects the R-RNA sequences which are similar in widely different organisms, can detect the presence or absence of any one or more of 'those organisms in a sample. A marked nucleic acid sequence, or sequences complementary to the R-RNA sequences similar in widely divergent organisms, can be used as such a probe in nucleic acid hybridization assay.

Because of the R-RNA sequences of closely related organisms are more similar than those of distantly related organisms, the method of my invention, which includes using a probe which detects only the R-RNA sequences which are similar in a particular narrow group of organisms, can detect the presence or absence of any one or more of those particular organisms in a sample, even in the presence of many non-related organisms. These group specific probes can be specific for a variety of different sized categories. One probe might be specific for a particular taxonomic genus, while another is specific for a particular family or another genus.

Group specific probes have the ability to hybridize to the R-RNA of one group or organisms but not hybridize to the R-RNA of any other group of organisms. Such a group specific complementary sequence will detect the presence of R-RNA from any member of that specific group of organisms even in the presence of a large amount of R-RNA from many organisms not belonging to that specific group.

The total number of R-RNA molecules in a sample is measured by using a marked sequence or sequences complementary to R-RNA and standard excess probe or excess sample RNA nucleic acid hybridization methodology.

The R-RNA content of cells from a wide variety of organisms is known in the art. In a broad group of similar organisms, for example bacteria, the amount of R-RNA per cell varies roughly 2-5 fold. Therefore, if the number of R-RNA molecules in a sample, and the broad class identity of the source of the R-RNA is known, then a good estimate of the number of cells present in the sample can be calculated. If the broad class identity is not known it can be determined by hybridizing the sample to a series of selected probes complementary to R-RNA, each of which is specific for a particular broad category of organisms.

At the present time, the operational detection and quantitation range of a single assay procedure is from 10⁴ R-RNA molecules (1 bacterium or 10⁻² mammailan cells) to about 10¹² R-RNA molecules (10⁸ bacteria or 10⁶ mammalian cells) a span of about 10⁸ in cell numbers. A single test could also be done in such a way as to operationally quantitate from 10³ bacteria to 10¹⁰ bacteria. The test is quite flexible in this way.

Because the test for R-RNA is specific and has the ability to detect the presence of very few organisms there is no need to amplify the numbers of organisms through a growth step.

The practice of that form of my invention which is directed to determining the presence of an organism which contains R-RNA, in a sample which might contain ' such organism, comprises basically:
a) bringing together the sample, or isolated nucleic acids contained in that sample, with a probe which comprises marked nucleic acid molecules which are complementary to the R-RNA of all organisms;
b) incubating the resulting mixture under pre-determined hybridization conditions for a predetermined time, and then;
c) assaying the resulting mixture for hybridization of the probe.

When my invention is directed to determining the presence of any member of a specific category of organisms which contain R-RNA in a sample which might contain such organisms, the method comprises:
a) contacting the sample, or the nucleic acids therein, with a probe comprising marked nucleic acid molecules which are complementary only to the R-RNA of members of the specific category or organisms, but not complementary to R-RNA from non-related organisms;
b) incubating the probe and the sample, or the isolated nucleic acids therein; and
c) assaying the incubated mixture for hybridization of said probe.

My invention can also be used to determine the number of organisms present in the sample under investigation, by adding to the assay in the second above described method in the event probe hybridization has occurred, the step of comparing the quantity of R-RNA present in the sample with the number of R-RNA molecules normally present in individual organisms belonging to the said specific group.

And, of course, included in the variations, within the scope of my invention, which can be used, is that which comprises, in lieu of the single probe of step (a) in the second of the above methods, a multiplicity or battery of different probes. In such a case, each separate probe comprises a marked nucleic acid molecule which is complementary only to the R-RNA of a specific group of organisms and each probe is specific for a different group of organisms; step (a) is followed by' incubating each probe-sample mixture under predetermined hybridization conditions for a pre-determined time, and then assaying each mixture for hybridization of the probe.

The method and means of my invention are more fully illustrated in the following description of characterizing features of test methods in accordance with the invention.

### Nucleic Acid Hybridization Test Procedures for Detecting and Quantitating RNA

A desirable detection test should: a) be rapid; b) be easy to use; c) be highly sensitive; and d) be able to detect and quantitate in just one lab assay.

The existence of a nucleic acid probe which will hybridize to R-RNA from any member of the genus Legionella, but does not hybridize to R-RNA from any other source, makes possible a rapid, easy to use, sensitive, in solution detection test which can both detect and quantitate, for example, Legionella bacteria with the performance of just one laboratory assay and does not require the purification of nucleic acids from the sample.

A description of the basic aspects of this in solution hybridization test procedure follows. While the procedure described is designed for detecting members of the genus Legionella, it is obvious that this same test procedure can be used with the appropriate probe to detect many other groups of organisms.

### Step 1. Preparing the Sample

Mix the sample with a solution containing a detergent and a proteolytic enzyme. The detergent lyses the bacteria 'and helps solubilize cellular components while the enzyme destroys the cellular proteins, including those enzymes which degrade RNA and DNA.. The composition of the detergent-enzyme mix depends upon the type of detergent and proteolytic enzyme used and the amount and type of sample to be checked. Detergents used include sodium lauryl sulfate, sarkosyl and Zwittergent, while the enzymes used include Proteinase K and Pronase. A wide variety of enzymes, solubilizing agents such as chaotropic agents, can be used. The probe can also be present in the detergent-enzyme mix added to the sample.

The enzyme-detergent acts very quickly on any Legionella bacteria in the sample. In most cases it is not necessary to incubate the mixture in order to make the R-RNA available for in solution hybridization with the probe. In certain cases a short incubation period is needed.

In other situations it is not necessary to include the proteolytic enzyme, and detergent alone will make the R-RNA available for in solution hybridization with the probe.

This approach provides a very rapid and easy method for getting the sample R-RNA into a state where it can hybridize with the probe in an in solution assay. In addition, it allows the hybridization to occur in solution without purifying the sample R-RNA. A key to this method is that the probe detects Legionella R-RNA. R-RNA is single stranded in the cell and ready to hybridize with the probe once the ribosomal proteins are removed from the R-RNA. In contrast, to directly detect the ribosomal R-RNA DNA (i.e., the gene for R-RNA) or any other DNA sequence it would be necessary to add a procedure which caused the double stranded R-RNA gene to separate into two single strands before the probe could hybridize to it.

To the best of my knowledge, there is no prior art concerning the use of an enzyme-detergent-sample method for making R-RNA or DNA available for in solution hybridization with a probe for the purpose of detecting and quantitating the presence or absence of organisms in general or a specific group of organisms.

### Step 2. Preparing the Hybridization Incubation Mixture

To the sample-enzyme-detergent mix add the probe and sufficient salt to enable hybridization to occur and incubate the resultant mixture at an appropriate temperature. The salt concentration and the temperature of hybridization incubation combine to determine the criterion. The criterion of the incubation condition must be equal to that used to select the probe or the specificity of the probe may change.

The incubation mixture must be incubated for a long enough time for hybridization to occur. The salt type and concentration determines the rate of hybridization which can be attained. Thus, certain salts will promote very rapid hybridization when used at the proper concentration. An example of such a salt is sodium phosphate. Legionella specific probe mixed with purified Legionella R-RNA in 3.0 M sodium phosphate buffer (pH = 6.8) (hereinafter termed PB) and incubated at 76° C hybridizes over 100 times more rapidly than the same amounts of Legionella probe and R-RNA incubated under standard conditions of 0.72 M NaCL, 76° C (these two conditions are equal in criterion). Other salts can also be used to effect this hybridization rate acceleration. These include most sodium, ammonium, rubidium, potassium, cesium, and lithium salts.

In 3 M PB at 76°C the hybridization rate of the Legionella specific probe with Legionella R-RNA present in the PB-enzyme-detergent-sample probe mixture is also accelerated by over 100 times over the hybridization rates seen for the standard incubation conditions. Hybridization also occurs between the probe and R-RNA in an enzyme-detergent-sample mixture under standard salt concentration conditions.

One of the features of the invention, as previously pointed out, is the ability to detect very small numbers of organisms by detecting their R-RNA. This is possible because of the large numbers of R-RNA molecules in each cell. In LegioneIla-like organisms 5,000 to 10,000 R-RNA molecules are present in each individual bacterial cell. One of the major determinants of the sensitivity of detection which can be achieved with nucleic acid hybridization is the rate of hybridization which can be attained. The combination of detection of R-RNA and the use of the rate accelerating incubation conditions described above make it possible to attain extremely high sensitivity of detection of bacteria and. other organisms in a very short period of time with the use of very small amounts of sample and probe. An illustrative example of this is described later.

To the best of my knowledge there is no prior art concerning the use of rate-accelerating systems with in solution hybridization tests for determining the presence or absence of an organism or group of organisms by detecting the R-RNA or DNA of the organisms of interest. There is also no prior art of which I am aware concerning the use of a combination of a rate-accelerating system and the enzyme-detergent-sample-probe mixtures to determine the presence or absence of a specific organism or group of organisms by detecting the R-RNA or DNA of the specific organism or group of organisms of interest.

### Step 3. Assaying the Incubation Mixture for Hybridization of the Probe with Target R-RNA

The signal that the sample contains the target R-RNA molecules (and therefore the target organism) is the presence of hybridized probe in the incubation mixture. Thus the incubation mixture must be assayed for the presence of hybridized probe at the end of the incubation period. It is desirable that such an assay be easy to perform and rapid. For this assay the incubation mix is processed by utilizing hydroxyapatite (HA). Under the proper conditions HA binds R-RNA rapidly and completely but does not bind the non-hybridized probe molecules. If a probe molecule is hybridized to a target R-RNA molecule the probe also binds to the HA because it is physically attached to the R-RNA.

Detection of organisms by detecting their R-RNA is a feature of the invention. The ability of the HA to bind R-RNA in seconds, while not binding the probe at all, has allowed the development of a hybridization assay method which takes minutes to perform, has great flexibility and which adapts well for handling multiple samples. In addition the sample-detergent-enzyme-probe incubation mixture, can be diluted into the appropriate buffer and directly processed to assay for the presence of hybridized probe.

HA is known in the art as a substance used for assaying hybridization of probes. The'assay method described here, which has great advantages over the prior art uses of HA (Brenner et al., Analytical Biochm (1969( 28 p. 477), can be carried out at room temperature and will work over a temperature range of about 15° C to about 90° C. It has fewer steps and does not require heating at each centrifugation step; it can be carried out in the presence or absence of detergents such as Zwittergent (Calbiochem, (San Diego, Calif.) and sodium lauryl sulfate. It is 3 - 5 times faster, and a single assay can be done in 3 - 5 minutes. It requires about 5 times less HA. Detergent concentration can range from 0 to 10%, while the phosphate concentration can range from 0.1 M to 0.2 M depending on the type of assay. The method can also be readily adapted for handling multiple samples.

Methods other than HA are available to assay for hybridization of the probe. These include enzyme assays such as the S₁ enzyme method, size separation methods, and a variety of sample immobilization methods. The probes discussed here can be used effectively with these and any other method of conducting hybridization and hybridization assays.

### Procedures for the Production of Group Specific R-RNA Probes

Different approaches can be used to produce group specific probes. All of these approaches but one rely on differential nucleic acid hybridization methods to identify and purify the group specific probe sequences.

### Procedure A:

The most useful procedure for producing group specific R-RNA probes uses recombinant DNA methodology. The steps involved in this procedure follow: (The specific details of standard DNA recombinant techniques are described in the book, Molecular Cloning, A Laboratory Manual, T. Maniatis et al., Cold Spring Harbor Publication (1982))
1. Isolate nucleic acid from a specific organism of interest. Standard isolation methods are used.
2. Using this isolated DNA, clone the R-RNA genes of this organism and then produce large amounts of the ribosomal gene DNA, using standard DNA recombinant technology, as shown in Maniatis et al., supra.
3. Reduce the R-RNA gene DNA to short pieces with restriction enzymes and make a library of these short DNA pieces, using standard DNA recombinant methods, as shown in Maniatis et al., supra.
4. Screen the library and identify a clone which contains a short R-RNA gene sequence which hybridizes only to R-RNA from other members of the taxonomic species of the organism of interest. Isolate this clone. It contains a species specific DNA sequence which is complementary only to the R-RNA of the specific species to which the organism of interest belongs.
   Screen the library further and identify and isolate the following clones: a) a clone which contains a DNA sequence complementary to R-RNA which will only hybridize to R-RNA from members of the taxonomic genus to which the organism of interest belongs; b) a clone which contains a DNA sequence complementary to R-RNA which will only hybridize to R-RNA from members of the taxonomic order to which the organism of interest belongs; c) a clone which contains a DNA sequence complementary to R-RNA which will hybridize only to R-RNA from members of the taxonomic family to which the organism of interest belongs; d) a clone which contains a DNA sequence complementary to R-RNA which will hybridize only to R-RNA from members of the taxonomic class to which the organism of interest belongs; and e) a clone which contains a DNA sequence complementary to R-RNA which will hybridize to R-RNA from as many different life forms as possible.
   The foregoing clone selection scheme is only one of a number of possible ones.
   Standard methods of cloning and screening are to be utilized, as discussed in Maniatis et al., supra.
5.
   a) Produce large amounts of each clone's DNA. From the DNA of each individual clone isolate and purify only the DNA. sequence which is complementary to R-RNA, using one of the many methods existing to accomplish this, e.g., as in Maniatis et al., supra.
   b) In certain instances the total DNA present in a clone is useful as a probe, in which case the total DNA isolated from the cloning vector is used.
   c) In certain other instances, the DNA single strand of the cloning vector which contains the DNA sequence complementary to R-RNA is used as a probe. In such case this strand must be isolated and purified, using one of the various methods which exist to accomplish this, as described by Maniatis et al.
6. The probe DNA'obtained in 5a, 5b, and 5c must be marked in some way so that it can be identified in the assay mixture. Many different kinds of markers can be used, the most frequently used marker being radioactivity. Others include fluorescence, enzymes, and biotin. Standard methods are used for marking the DNA, as set out in Maniatis et al., supra.
7. The group specific R-RNA gene sequence in the cloning vector exists in a double strand state. One of these strands is complementary to R-RNA and will hybridize with it. The other strand will not hybridize to R-RNA but can be used to produce. marked group specific sequences complementary to R-RNA. This is done by utilizing a DNA or RNA polymerase and nucleic acid precursor molecules which are marked. The enzyme will utilize the marked precursors for synthesizing DNA or RNA using the DNA strand as a template. The newly synthesized marked molecule will be complementary to R-RNA and can be used as a group 'specific probe. The template DNA can be removed by various established means leaving only single strand marked nucleic acid, as described in Maniatis, et al., supra, and the article by Taylor et al., in Biochemica and Biophys. Acta (1976) 442, p. 324.

### Procedure B:

Several enzymes can utilize R-RNA from any source as a template for the synthesizing of marked DNA complementary to the entire R-RNA sequence. Group specific sequences complementary only to the R-RNA of a particular class of organisms can be isolated by a hybridization selection process. The fraction of the synthesized marked DNA which hybridizes only to the R-RNA from members of a specific class of organisms can be isolated by standard hybridization procedures. An example of this process is presented hereinafter. Such a probe can be produced in sufficient quantities to clone as is described in A.
The base sequence of this clone can be determined by standard methods and the sequence used to direct the production of the probe by chemical synthesis using standard methods.

### Procedure C:

The nucleotide sequences of R-RNA from widely different organisms have been determined. Group specific sequences similar to a specific group of organisms can be identified by comparing these known sequences. A sequence complementary to this group specific R-RNA sequence can then be chemically synthesized and marked, using standard methodology.

### Isolating Sample Nucleic Acid

Standard prior art methods can be used to isolate nucleic acid from the samples to be assayed. One standard method of nucleic acid isolation and purification is presented in the examples section and is also discussed in Maniatas et al., supra.

A new technique for making nucleic acids available for in solution hybridization without performing e purification step is described hereinafter.

### Performing the Nucleic Acid Hybridization

An appropriate amount of marked probe is mixed with the sample nucleic acid. This mixture is then adjusted to a specific salt concentration (NaCl is usually used) and the entire mix incubated at a specific temperature for a specific time period. At the end of the time period the mixture is analyzed by performing a hybridization assay. Many different combinations of salt, solvent, nucelic acid concentrations, volumes, and temperatures exist which allow nucleic acid hybridization. The preferred combination depending on the circumstances of the assay. It is important, however, that the criterion (see "Definitions) of the hybridization steps be identical to criteria used to identify and select the group probe. If the criteria of the hybridization step is different, the probe specificity may change. See: "Repeated Sequences in DNA", by Britten and Kohne, Science (1968) 161 p. 529; "Kinetics of Renaturation of DNA", by Wetmur and Davidson, J. Mol. Biol. (1968) 31 p. 349; "Hydroxyapatite Techniques for Nucleic Acid Reassociation", by Kohne and Britten; Procedures in Nucleic Acid Research (1971), eds. Cantoni and Davies, Harper and Row, Vol 2, p. 500.

Two different approaches are used with regard to the amount of probe and sample nucleic acid present in the hybridization mixture. In one, the excess probe method, there is more probe present than sample nucleic acid, in this case RNA. With the other, the excess RNA method, there is more R-RNA present than probe. ,The excess probe method is the method of choice for detecting the presence of RNA in unknown samples. It has several advantages which are discussed below. See Tables 1 and 2 for further discussion of these two approaches.

Using the excess probe method, the detection and quantitation can be done with just one lab assay point, if the proper KNA probe is available. If the hybridization has gone to completion the amount of probe which has hybridized is a direct measure of the amount of RNA present in the sample. The fact that the probe hybridizes at all indicates that RNA is present, and the amount of probe which hybridizes indicates the amount of RNA present in the sample.

Making sure that the hybridization has gone to completion in a known time is important in order to quantitate the RNA. This is readily done by adding enough probe to ensure that the hybridization goes to completion in a selected time period. The more probe added, the faster completion is reached. Thus the excess probe method provides a means to ensure that the hybridization has gone to completion and to know when this has occurred.

In contrast, the detection and quantitation of RNA can't be done with one lab assay point when using the excess R-RNA method. In addition, the time when the test point should be taken cannot be predicted in the excess RNA method. Unknown samples with small amounts of RNA will hybridize much more slowly than samples with large amounts of RNA.

### The Assay for Hybridization

The signal that RNA of the specific group is in the sample is the presence of double strand marked probe. Many different methods, well documented in the literature, are available for assaying the hybridization mixture for the presence of marked probe in the double strand form. The choice of method depends upon the method chosen for the hybridization step, the composition of the hybridization mixture, the type of marker on the probe and other factors. One commonly used method is described hereinafter. See also Wetmur and Davidson, Kohne and Britten, and Thomas et al., supra. Also the article by Flavell et al., Eur. J. Biochem. (1974) 47 p. 535. And also, the article by Maxwell et al., Nucleic Acids Research (1978) 5 p. 2033.

In all cases, however, it is improtant to either assay at or above the same criterion used for the hybridization reaction or at a criterion at which hybridization cannot occur.

### Quantitation of Nucleic Acid Sequences by Nucleic Acid Hybridization

The quantity of nucleic acid present in a sample can be determined in several ways by nucleic acid hybridization, using methods well known to the art. The two methods are disclosed hereinafter using the example of quantitating R-RNA.

It will be understood. that the present method is generally applicable in any case where it is necessary to determine the presence or absence of organisms which contain RNA or DNA and that such includes biological samples such as sputum, serum, tissue swabs, and other animal fluids and tissues as well as industrial and pharmaceutical samples and water. Specific details of the approach will change depending on whether RNA or DNA is being quantitated but the general approach is the same for both DNA and RNA.

**TABLE 1**

| EXCESS SELECTED PROBE METHOD | | | | |
|---|---|---|---|---|
| PROBE: | The probe is a specific, selected, marked sequence from a member of bacteria group B, which represents 10 percent of the base sequence of the R-RNA, and hybridizes' completely with R-RNA from group B bacteria, but does not hybridize with R-RNA from other organisms. The probe cannot hybridize with itself. | | | |
| A. | Positive Homologous Control 0.1 microgram Probe + 10⁻³ micrograms Sample group B R-RNA | Hybridize to completion and assay for double strand probe | a) | One percent of the probe will form double strand molecules. |
| | | | b) | This is a direct measure of the R-RNA sample. The number of probe molecules hybridized equals the number of R-RNA molecules present. |
| B. | Heterologous Control 0.1 micrograms Probe + 10⁻³ micrograms Sample human R-RNA | Hybridize to completion and assay for double strand probe | | The probe does not hybridize with any R-RNA but R-RNA from group B bacteria |
| C. | Unknown Sample 0.1 micrograms Probe + Unknown Unknown Sample | Hybridize to completion and assay for double strand probe | a) | If no group B R-RNA is present, no probe will hybridize. |
| | | | b) | If group B R-RNA is present, the probe will hybridize and form double strand molecules. |
| | | | c) | The number of probe molecules hybridized equals the number of group B R-RNA molecules present in the sample. |
| | | | d) | If one percent of the probe hybridizes, group B R-RNA is present since the probe was selected so that it would hybridize only with R-RNA from a group B bacteria. Since the probe will only hybridize to group B R-RNA, the presence of other R-RNAs will not interfere with the detection or the quantitation of. any bacterial R-RNA present. |
| | | | e) | Using a selected probe makes it easier to ensure that the hybridization is complete. A selected probe representing 10 percent of the R-RNA sequence will hybridize 10 times faster than a probe which is representative of the total R-RNA sequence. |
| | | | f) | The detection of R-RNA in general is not possible since the probe hybridizes only with group B R-RNA. The sensitivity of detection of group B R-RNA is extremely high. |

| D. | Summary | | | |
|---|---|---|---|---|
| | The excess probe method needs just one assay point in order to detect and quantitate group B organisms. | | | |

**TABLE 2**

| EXCESS R-RNA METHOD: THE USE OF A SELECTED PROBE | | | | |
|---|---|---|---|---|
| PROBE: | The probe is specific, selected, marked sequence from group B bacteria, which represents one-tenth of the R-RNA base sequence of one member of group B. The probe hybridizes completely with R-RNA from group B, but does not hybridize to R-RNA from other organisms. The probe cannot hybridize with itself. | | | |
| A. | Positive Homologous Control Sample strand 0.1 micrograms Group B R-RNA + 10⁻³ micrograms Probe | Hybridize to completion and assay for double probe . | a) | The fraction of probe which hybridizes is a direct measure of the similarity between the R-RNA and the probe. In this case 100 percent of the probe can hybridize. |
| | | | b) | This percentage is not a measure of the amount of R-RNA present. In order to determine this the kinetics of the reaction must be determined. |
| B. | Heterologous Control Sample 0.1 micrograms human R-RNA + Probe 10⁻³ micrograms | Hybridize to completion and assay for double strand probe. | | The probe does not hybridize to non-bacterial R-RNAs. |
| C. | Unknown Sample Sample + Probe 10⁻³ micrograms | Hybridize to completion and assay for double strand probe. | a) | If no group B R-RNA is present in the sample there will be no hybridized probe. |
| | | | b) | If group B R-RNA is present the probe will be hybridized. |
| | | | c) | The amount of R-RNA can't be determined from the percentage hybridization at the completion of the reaction. In order to determine this the kinetics of the hybridization must be determined. Since the probe will hybridize with only one type of R-RNA, the kinetic determination is simple. |
| | | | d) | If 100 percent of the probe has hybridized with the sample, this means that group B R-RNA is present in the sample. It does not indicate that only this R-RNA is present. Other R-RNAs which do not hybridize with the probe may also be present in the sample. |
| | | | e) | If 100 percent of the probe hybridizes with the sample, it is possible to specifically quantitate the group B R-RNA in the presence of human R-RNA by determining the kinetics of hybridization of the probe with the sample R-RNA. Since the probe will hybridize only with group B R-RNA such a kinetic reaction will have only one component, the one from reacting with group B R-RNA. |
| | | | f) | There are situations where the hybridization can't go to completion. In this method the sample R-RNA must drive the hybridization to completion, since only a very small amount of probe is present. If there is not sufficient R-RNA in the sample, the hybridization will not be completed. The interpretation of such a situation is discussed below. |
| | | | | If hybridization of unknown sample results in 20 percent hybridization of the probe at the usual assay time, it is not possible to tell if the reaction is complete with only one time-point. It is necessary to take another point at double the original time to determine if the hybridization value increases. If it does not increase then the hybridization is complete. In this case the R-RNA is at such low concentration in the sample that the probe is in excess, and the number of R-RNA molecules present in the sample is equal to the number of probe molecules hybridized. |
| | | | | If the hybridization value is increased, the hybridization was not over at the first time-point. A third time-point must then be done to determine whether the reaction was over at the second time point. |

| | Summary | | | |
|---|---|---|---|---|
| D. | The excess sample R-RNA method needs multiple assay points in order to detect and quantitate, and is much more time-consuming than the excess probe method. | | | |

### USE OF SELECTED PROBES COMPLEMENTARY TO ONLY A PARTICULAR FRACTION OF THE R-RNA SEQUENCE FROM A PARTICULAR·SOURCE TO DETECT R-RNA VERSUS USE OF UNSELECTED PROBES COMPLEMENTARY TO THE ENTIRE R-RNA SEQUENCE FROM A PARTICULAR SOURCE TO DETECT R-RNA

One aspect of my invention, which comprises using specifically selected probes complementary to only a particular fraction of the R-RNA sequences to detect, quantitate, and identify R-RNA has important capabilities and advantages over using unselected probes or sequences complementary to the entire R-RNA sequence to detect R-RNA. The advantages of using a selected probe in both excess R-RNA and excess probe hybridization methodologies are set forth below. The problems with using a completely representative probe are also presented.

The advantages of using a selected probe over using a completely representative R-RNA probe, with excess probe hybridization, as well as with excess R-RNA hybridization, is set out below:

| Advantages of the Excess Probe Hybridization Method | |
|---|---|
| Problems with Completely Representative R-RNA Probe | Advantages of Using Selected Probes |
| 1. R-RNA can be detected in a sample with the excess probe method but there is no way of determining the type of R-RNA present. Thus this probe can't be used to specifically detect and quantitate the presence of a particular R-RNA in an unknown sample, with the excess probe hybridized method. | The selected probe can be used to sensitively and specifically detect and quantitate the presence of a particular R-RNA, in an unknown sample when used in an excess probe hybridization method. This can be done with just one lab assay, even in the presence of R-RNA from other organisms. |
| 2. As stated above, the excess probe method cannot be used with this probe to detect or quantitate the presence of a particular R-RNA in a sample. For this purpose the probe must be used in the excess R-RNA method. | The use of a selected probe makes it possible to use the excess probe method for detecting and quantitating the presence of a particular R-RNA in an unknown sample. This greatly simplifies the task. |
| The excess R-RNA method is much more time consuming, requires much more work, and is much more complicated than the excess probe method. | |

| Advantages of the Excess R-RNA Hybridization Method | |
|---|---|
| Problems with Completely Representative Probe | Advantages of Using Selected Probe |
| 1. R-RNA can be detected in an unknown sample with this probe, but in many cases there is no way of determining the type or quantity of R-RNA which is present. Thus in many instances the probe cannot be used to specifically detect and quantitate the presence of a particular R-RNA in an unknown sample. | The selected probe can be used to specifically detect and quantitate the presence of a particular R-RNA in an unknown sample in all situations. This can be done even in the presence of large amounts of R-RNA from other organisms. |
| 2. In many cases the sensitivity of detection of a specific R-RNA is limited by the presence of R-RNA from other organisms. | With the selected probe the presence of R-RNA from other organisms does not lower the sensitivity of detection of a particular R-RNA. |
| 3. In many cases where it is possible to detect and quantitate the presence of particular R-RNA, it requires a lot of work. | The detection and quantitation of the presence of a particular R-RNA is much easier when a selected probe is utilized. |

### Illustrative Embodiments

My invention, illustratively, may be used to determine whether a sample contains any member of a particular group of living organisms. The method, described in the following examples, is a test which may be used'to detect and quantiate the presence of any member or members of a particular group of bacteria in a sample, even in the presence of large numbers of organisms which are not members of that particular group.

As set forth in the examples, applicant's method involves first producing a group specific R-RNA probe which, at a specific criterion, hybridizes to R-RNA from any member of the specific group of interest, but does not hybridize to R-RNA from any other organims. The use of such a probe in a nucleic acid hybridization test allows the detection of any member of that specific group, even in the presence of large numbers of other organisms.

Examples of the practice of the invention are listed later. Each example involves the production of a marked nucleic acid probe which will hybridize only with R-RNA from members of a particular group of organisms.

The basic outline of the method used to produce each probe is as follows:
1. Produce marked nucleic acid complementary to the R-RNA of a member of the group of interest.
2. Hybridize this DEA to R-RNA from a member of the group or groups of organisms evolutionarily most closely related to the group of organisms for which the probe is to be specific. Select the fraction of the marked nucleic acid which, at a specific criterion, does not hybridize to R-RNA from a member of this closest related group of organisms. This fraction is specific for the R-RNA of the organism group of interest and does not hybridize with R-RNA from the most closely related group or groups or any other organism.

### Example 1: Production of Probe Which Will Hybridize to R-RNA.from any Bacteria

In a typical situation, about 10⁶ -10⁷ mammalian cells are grown in a tissue culture plate at one time. Bacterial species, especially members of the taxonomic class Mollicutes, are known to contaminate tissue culture cells. Members of the class Mollicutes, unlike most other bacteria, are not readily eliminated by antibiotics, and are troublesome contaminants of cell cultures. Many different Mollicutes species have been detected in tissue culture cells. If just one of these organisms is present in the culture plate, it has the potential, even in the presence of antibiotics, to multiply and produce hundreds of organisms per cell. Such organisms are capable of altering the activity of cells, thereby affecting the results of various studies and the marketability of cell culture products.

Prior art methods for detecting these organisms involve basically qualitative tests, the most commonly used being growth tests, differential staining tests and immunologic assays. The growth tests, while quite sensitive, take 3 - 6 weeks. They have the additional disadvantage that many organisms are difficult or impossible to grow.

While the actual detection sensitivity of the staining method is not known, it is known that more than several organisms per cell have to be present.

Immunologic tests are qualitative tests and involve using antibody toward a particular species. While they can be carried out rapidly, they are not very sensitive; furthermore, many different antibodies would be required to detect all types of Mollicutes.

The embodiment of applicant's method described below, is a test which may be used to detect and quantitate the presence of any member of the group of all bacteria, including the taxonomic class Mollicutes, to detect the presence of Mollicutes in tissue culture, to detect the presence of bacteria in tissue which is normally free of bacteria, and to detect the presence of the bacteria even in the presence of large numbers of mammalian cells.

As set forth in the example, applicant's method involves first making a specific R-RNA probe which is complementary to R-RNA from any bacteria but is not complementary to mammalian cell R-RNA. The use of such a probe in a nucleic acid hybridization test allows the detection of any bacteria type, even. in the presence of large numbers of mammalian cells.

A detailed description of this embodiment of the invention follows:

### Preparation of R-RNA from Mammalian and Bacterial Cells

Mammalian cells are resuspended in 0.3 M NaCl, 0.02 M Tris, pH = 7.4. Sarkosyl is added to a final concentration of 1 percent to lyse the cells. Immediately upon lysis an equal volume of a 1/1 mixture of phenol/ chloroform is added and the resulting mixture shaken vigorously for 2 minutes. The mixture is then centrifuged (8000 x g for 10 minutes) to separate the aqueous and organic phases. The aqueous phase is recovered, and to this is added another volume of phenol/chloroform. After shaking and centrifugation as above, the aqueous phase is again recovered. To this is added 2 volumes of 95% ethanol and this mixture is placed at -20° C for 2 hours to facilitate precipitation of the nucleic acids. Then the mixture is centrifuged (8000 x g, 10 minutes) in order to sediment the precipitate to the bottom of the tube. The liquid is then removed. The pelleted nucleic acid is redissolved in water. This solution is then made to 0.2 m NaCl, 5 x 10⁻³ m MgCl₂, 5 x 10⁻³ M CaCl₂. 0.02 M Tris (pH = 7.4), 50 micrograms per ml of deoxyribonuclease I and incubated at 37° C for 1 hour. Then add an equal volume of phenol/chloroform and shake as above. Centrifuge as above and recover the aqueous phase. Ethanol precipate the RNA as above. Centrifuge the precipitate as above and redissolve the pelleted RNA in water. Make this solution to 2 M LiCl and place it at 4° C for 10 - 20 hours in order to facilitate the precipitation of the high molecular weight RNA. Then centriguge this solution to collect the precipate and redissolve the precipitate in water. This preparation of RNA contains greater than 95% R-RNA.

Bacterial R-RNA is isolated in a similar manner with the following exceptions. In those cases where detergent alone does not lyse the bacteria, other means are employed. This usually involves pretreating the bacteria with an enzyme (lysozyme) to make them susceptible to lysis by sarkosyl. After lysis of the bacteria the isolation procedure is as described above.

Purified R-RNA is stored at -70° C.

### Production of Radioactive DNA Complementary (³H-cDNA) to Mollicutes R-RNA

R-RNA from the species Mycoplasma hominis (M. hominis), a member of the taxonomic class Mollicutes, is used as a template to synthesize radioactive cDNA complementary to M. hominis R-RNA.

This cDNA is produced by utilizing the ability of an enzyme, reverse transcriptase, to utilize R-RNA as a template and produce ³H-cDNA complementary to R-RNA. The reverse transcriptase reaction mixture contains the following: 50 mM Tris·HCL (pH = 8.3), 8 mM MgCl₂, 0.4 mM dithiothreitol, 50 mM KCL, 0.1 mM ³H-deoxythymidinetriphosphate (50 curies per millimole), 0.2 mM deoxyadenosinetriphosphate, 0.2 mM deoxycytidinetriphosphate, 0.2 mM deoxyguanosinetriphosphate, 200 micrograms per ml of oligodeoxyribonucleotide primer made from E. coli DNA, 50 micrograms per ml of M. hominis R-RNA and 50 units per ml of AMV reverse transciptase. This mixture is incubated at 40° C for 30 minutes. Then ethylene diamine tetraacetic acid (EDTA) (pH = 7.3), sodium dodecyl sulfate (SDS), NaCl and glycogen are added to final concentrations of 10⁻²M, 1 percent, 0.3 M, and 100 micrograms per ml respectively. The solution is then mixed with 1 volume of phenol/chloroform (1/1) and shaken vigorously for 2 minutes, then centrifuged (8000 x g for 10 minutes) and the aqueous phase recovered. The nucleic acids are precipitated by the addition-of 2.5 volumes of 95% ethanol. The precipitate is recovered by centrifugation and redissolved in H₂O: This solution contains the template R-RNA and the newly synthesized ³H-cDNA.

This solution is then, made to 0.3 M NaOH and incubated at 50° C for 45 minutes, and cooled in ice and neutralized with 0.3 M HCl. Two and one-half volumes of 95% EtOH are then added to precipitate the remaining nucleic acid and the resulting precipitate redissolved in water. This solution is then passed over a Sephadex G-100 column equilibrated to 0.3 M NaCl, 0.1 percent sarkosyl and the excluded volume recovered. This solution is ethanol precipitated and the resulting precipitate redissolved in a small volume of water. The process described in this paragraph removes the template R-RNA and any remaining precursor material from the ³H-cDNA preparation.

The ³H-cDNA is then hybridized to M. hominis R-RNA to ensure that it is indeed complementary to this R-RNA. The hybridization mixture consists of 0.05 micrograms of single strand ³H-cDNA, 20 micrograms of M. hominis R-RNA, and 0.48 M PB (phosphate buffer) in 1 ml. This mixture is incubated for 0.2 hours at 65° C and is then diluted to 0.14 M PB and passed over a hydroxyapatite (HA) column equilibrated to 0.14 M PB and 65° C. ³H-cDNA hybridized to R-RNA absorbs to the hydroxyapatite (HA) column while non-hybridized ³H-cDNA passes through the column. The hybridized ³H-cDNA is then recovered by elution of the HA column with 0.3 M PB. This fraction is then dialysed to remove the PB, ethanol precipitated to concentrate the nucleic acid, centrifuged and the nucleic acid redissolved in water. This solution is then treated with NaOH as described above in order to remove the R-RNA. After neutralization, addition of glycogen carrier and concentration by ethanol precipitation, the ³H-cDNA is redissolved in a small volume of water. This solution contains only ³H-cDNA which is complementary to M. hominis R-RNA.

### Selection of ³H-cDNA Which is Complementary to M. hominis RNA but is not Complementary to Human R-RNA

The purified ³H-cDNA is further fractionated by hybridizing it with a great excess of human R-RNA. The hybridization mixture consists of 0.05 micrograms of H-cDNA, and 40 micrograms of human R-RNA in one ml of 0.48 M PB. This is incubated at 68° C for 1 hour and the mixture is then diluted to 0.14 M PB and passed over HA equilibrated to 55° C and 0.14 M PB. The fraction (about 50% of the total) which does not adsorb to the HA (i.e., ³H-cDNA not hybridized to human R-RNA) is collected. This fraction is repassed over a new HA column under the same conditions. Again the non-adsorbed fraction is collected. This fraction is dialysed to remove the PB, ethanol precipitated to concentrate the nucelic acid and redissolved in water. This solution is treated with NaOH, as described earlier, in order to remove the human R-RNA. After neutralization, addition of glycogen carrier, and concentration by ethanol precipitation, the ³H-cDNA is redissolved in a small volume of water. This ³H-cDNA preparation is complementary to M. hominis R-RNA but is not complementary to human R-RNA.

### Hybridization of Selected ³H cDNA with R-RNA from Different Source

The production of the selected ³H-cDNA probe allows the detection of bacteria, including members of the class Mollicutes in mammalian tissue culture cells and mammalian tissues by detecting the presence of bacterial R-RNA by nucleic acid hybridization. A necessary requirement of such a test is that the selected probe must not hybridize to R-RNA from mammalian cells which do not contain bacteria. That this requirement is met is shown in Table 3V

Table 3, parts II and III shown that the probe will detect all members of the class Mollicutes and should detect all types of bacteria. For example, Legionella pneumophe . and E. coli and Bacillus subtilis are representatives of very different bacterial types and the probe hybridizes with R-RNA from each of these types. Evolutionary considerations indicate that this probe will hybridize to R-RNA from virtually any known or unknown bacteria. This is due to the extreme conservation of the R-RNA nucleotide sequence during evolution.

This selected probe is useful for detecting the presence of a specific class of bacteria, Mollicutes, in tissue culture cells. In most tissue culture cells antibiotics are present in the growth medium and this prevents the growth of virtually all bacteria but members of the class Mollicutes. Thus any contamination of a tissue culture preparation is almost certain to be due to a member of the class Mollicutes.

An important aspect is the ability to determine the number of organisms present. In most cases, cell lines and their products are discarded when cells are shown, by prior art methods, to be contaminated. The ability to quantitate these organisms makes it possible to make judgments as to the severity of any effects due to contamination. The degree of a contamination may be very light, and only one organism per 1000 cells present. This level of contamination would have very little effect on the cells and in many situations the cell products need not be discarded. The decision might be made to retain valuable cell lines until they become more heavily contaminated. Quantatitive considerations are important for judging the importance of any kind of a bacterial contamination.

**TABLE 3**

| Hybridization of Selected Mollicutes ³H-cDNA with R-RNA from Widely Different Sources | | | | | |
|---|---|---|---|---|---|
| | | Source of R-RNA | | | Percent Hybridization of ³H-CDNA with R-RNA |
| I. | Control Experiments | A. | No R-RNA added, Self Reaction of ³H-cDNA | | < 1% |
| | | B. | Mock R-RNA isolation | | < 1% |
| | | C. | Human cell RNA known to be contaminated with M. hominis R-RNA | | 97% |
| II. | Hybridization of ³H-cDNA with R-RNA from different species of taxonomic class Mollicutes | A. | Members of the order of Mycoplasmatales | | |
| | | | 1. | Mycoplasma hominis (infects humans) 97% | |
| | | | 2. | Mycoplasma salivarius (infects humans) | 93% |
| | | | 3. | Mycoplasma hyorhinis (infects pigs) | 84% |
| | | | 4. | Mycoplasma pulmonis (infects mice) | 82% |
| | | B. | Members of the Order Acholeplasmataceae | | |
| | | | 1. | Acholeplasma laidlawii isolate #1 (infects cows, birds, dogs, house cats, mice, sheep, pigs, and primates) | 52% |
| II. | Cont'd | | 2. | Acholeplasma laidlawii (isolate #2) | 53 % |
| | | C. | Members of the Order Spiroplasma- taceae | | |
| | | | 1. | SMCA (infects insects and mice) | 69 % |
| | | | 2. | Honey bee (isolated from honey bee) | 68% |
| | | | 3. | Cactus (isolated from cactus) | 71 % |
| | | | 4. | Corn Stunt (isolated from corn) | 69 % |
| | | | 5. | Corn Stunt-(isolated from insect) | 65 % |
| III. | Hybridization of ³H-cDNA with R-RNA from other types of bacteria (taxonomic Class Schizomytes) | | A. | Member of the Family Enterobacteraceae | |
| | | | 1. | Escherischia coli (infects mammals) | 52 % |
| | | | B. | Member of the Family Legionellaceae | |
| | | | 1. | Legionella pneumophila (infects man) | > 28 % |
| III. | Cont'd | C. | Member of the Family Micrococcaceae | | |
| | | | 1. | Micrococcus luteus | 50-60 % |
| | | | 2. | Staphylococcus aureus | 50 % |
| | | D. | Member of the Family Lactobacillaceae | | |
| | | | 1. | Streptococcus faecalis | 50 % |
| | | E. | Member of the Family Bacillaceae | | |
| | | | 1. | Bacillus subtilis | 40 % |
| IV. | Hybridization of ³H-cDNA with R-RNA Yeast | | | | 2 % |
| V. | Hybridization of ³H-cDNA with R-RNA from mammals and a bird | | | Human (primate) | 1 % |
| | | | | Cow (bovine) | 1 % |
| | | | | Mouse (rodent) | 1 % |
| | | | | Rat (rodent) | 1 % |
| | | | | Hamster (rodent) | 1 % |
| | | | | Rabbit (lagomorph) | 1 % |
| | | | | Chicken (avian) | 1 % |

Excess R-RNA hybridizations are done at 68° C, 0.48 M PB. Hybridization assays are done with hydroxyapatite at 67° C in 0.14 M PB, 0.005% sodium dodecyl sulfate. The hybridization exposure is sufficient to ensure complete reaction of the ³H-cDNA with nuclear R-RNA or for mitochondrial R-RNA. Non-bacterial R-RNA Cot's of at least 2 x 10³ are reached in the case of the mammals and bird. A non-specific signal of 1-2 percent has been substracted from the hybridization values presented above.

### Quantitation of R-RNA by Nucleic Acid Hybridization

The amount of bacterial R-RNA present in a sample can be determined by measuring the kinetics of hybridization of the selected ³H-cDNA probe with the RNA isolated from a tissue and comparing these kinetics to those of a known standard mixture. This can be done even in the presence of a large excess of mammalian cell R-RNA since the probe does not hybridize with this R-RNA (see Table 3V).

For measuring the kinetics, the hybridization mixtures contain, 10⁻⁵ to 10⁻⁴ micrograms of ³H-cDNA and 1 to 10³ micrograms of purified sample RNA in 0.01 to 0.1 ml of 0.48 M PB. This mixture is incubated at 68° C and aliquots are removed, diluted to 0.14 M PB and assayed for hybridization at various times after the initiation of the reaction. Hybridization assays are performed using hydroxyapatite as described earlier. The results obtained are compared to the hybridization of the probe reacted with standard RNAs containing known amounts of bacterial R-RNA. These standards are mixtures of mammalian cell RNA and known amounts of a specific bacterial R-RNA.

### Detection and Quantitation of Members of the Class Mollicutes in Tissue Culture Cells

Table 4 presents data obtained by hybridizing the selected probe with RNA isolated (as described earlier) from three different tissue culture cell sampels. Only cell line number 3 is detectably contaminated and the kinetics of the reaction indicated that about 5 x 10⁷ bacterial cells are present in the tissue culture cells.

**TABLE 4**

| Detection and Quantitation of Mollicutes in Tissue Culture Cells | | | |
|---|---|---|---|
| Cell Line | Hybridization Time (hours) | Percent Hybridization of³H-cDNA with RNA | Number of Bacteria Detected |
| 1. 44-2C (rat) | 17 | 1 | None detected |
| | 40 | 1 | None detected |
| 2. P388 D1M (mouse) | 1.1 | 1 | None detected |
| | 22.5 | 1 | None detected |
| 3. P388 D1C (mouse) | 0.025 | 20 | 5 x 10⁷ |
| | 16.2 | 78 | (about 1 Mollicute per mammalian in cell) |

Excess R-RNA Hybridizations are done at 68° C in 0.48 M PB in a volume of 0.01 to 0.04 ml. Each mixture contains 2 x 10⁵ micrograms of ³H-cDNA probe and 50 - 200 micrograms of sample RNA.

The following example is another embodiment of the method of my invention, used for detecting very small numbers, even one trypanosome, in the presence of a large number of blood cells.

The detection of trypanosomes is important since certain members of the protozoan group Trypanosoma are pathogenic for humans, causing diseases that include East African sleeping sickness, West African sleeping sickness, and South American trypanosomiasis. These organisms are large and have varying characteristic shapes, depending on the stage of the life cycle. Prior art methods rely mainly on serologic, differential staining coupled with microscopic examination and animal inoculation procedures for detecting these organisms in humans. The serodiagnostic methods vary in sensitivity and specificity and may be difficult to interpret. The microscopic methods are most used, however small numbers of the trypanosomes are cften difficult to detect in the presence of large numbers of blood cells. Animal inoculation is a long and costly procedure.

The embodiment of the invention set forth in the following example is a method which makes it relatively easy to detect the presence of one trypanosome even when co-present with a large number of blood cells.

### Example 2

### Production of Radioactive DNA Complementary to Trypanosome R-RNA

Radioactive DNA complementary (³H-cDNA to trypanosoma brucei R-RNA is produced in the same way as M. hominis ³E-cDNA, which·is described above in detail, execpt that Trypanosoma brucei R-RNA is used as a template.

### Selection of Trypanosome ³H-cDNA Which is Complementary to Trypanosome R-RNA but is not Complementary to Human R-RNA

This is done in the same way as described earlier for M. hominis except that Trypanosomabruce, ³H-cDNA is hybridized to the human R-RNA.

### Use. of Selected Trypanosome ³H-cDNA to Detect and Quantitate Trypanosomes in Human Tissue or Fluid

The production of the selected ³H-cDNA probe allows the detection and quantitation of trypanosomes in human samples by detecting the presence of trypanosome R-RNA. A necessary requirement of such a test is that the selected probe must not hybridize to R-RNA from human cells which do not contain trypanosomes. Table 5 shows that this requirement is met.

**TABLE 5**

| Hybridization of Selected Trypanosoma brucei ³H-cDNA with R-RNA from Different Sources | |
|---|---|
| R-RNA Source | Percent Hybridization of ³H-cDNA with R-RNA |
| No RNA added | 1 % |
| Trypanosoma brucei R-RNA | 98 % |
| Bacterial (Mycoplasma hominis) R-RNA | 1 % |
| Human R-RNA | 1 % |
| Human R-RNA known to be contaminated with Trypanosame brucei | 98 % |

Excess R-RNA hybridizations are done at 65° C in 0.48 M PB. Reactions are run for 24 hours and the hybridization exposure is sufficient to ensure complete reaction of the human nuclear or mitochondrial R-RNAs and the bacterial R-RNA. Hybridization assays are done with hydroxyapatite at 72° C in 0.14 M PB, 0.005% sodium dodecyl sulfate.

One illustrative probe which I have prepared is specific only for members of the genus Legionella. The probe hybridizes to greater than fifty percent with nucleic acids from diverse members of the genus Legionella, and does not hybridize significantly with nucleic acids from mammals, yeast and a variety of widely diverse bacterial strains (Table 8). The probe hybridizes well even with Legionella species such as L. pneumophila and L. micdadei which show little or no bulk DNA relatedness. Other known Legionella species can be detected by this probe used in Table 6 as listed in Table 7. All of the known Legionella species (thus far 23 different species) have been examined and all can be specifically detected with the probe used in Table 6.

The specificity of this probe makes it possible to detect and quantitate the presence of Legionella species, even in the presence of large numbers of non-related bacterial or mammaliam cells. Thus, liver cells from a L. pneumophila infected hamster were assayed for the presence and number of Legionella organisms by using the specific probe and well established nucleic acid hybridization procedures. The liver had previously been assayed by a microbiological growth test which indicated that about 10⁷ Legionella organisms per gram were present in the infected liver. Nucleic acid hybridization analysis indicated about 1 - 2 x 10⁸ Legionella organisms per gram of liver. These results suggest that the plating efficiency in the growth test is about 5 - 10 percent.

The specific probe allows the highly sensitive and rapid detection of Legionella organisms even in the presence of large numbers of mammalian cells. In an assay which took less than 1 day the probe easily detected the presence of about 400 Legionella organisms which were mixed with 0.4 mg of liver (about 2 x 10⁵ cells).

**TABLE 6**

| Hybridization of Legionella Specific Probe With Nucleic Acids from Widely Different Sources | | | | |
|---|---|---|---|---|
| | | Nucleic Acid Source | | Normalized Percent Probe Hybridized |
| I. | Controls | 1) | No nucleic acid | 1 |
| | | 2) | Mock nucleic acid Isolation | 1 |
| | | | 3) L. pneumophila infected hamster tissue | 100 (Actual percent = 81) |
| II. | Legionellaceae | 1). | L. bozemanii (WIGA) | > 50 |
| | | 2) | L. dumoffii (TEX-KL) | >50 |
| | | 3) | L. garmanii (LS-13) | >50. |
| | | 4) | L. jordanis (BL540) | >50 |
| | | 5) | L. longbeachae | >50 |
| | | 6) | L. micdadai (HEBA) | >50 |
| | | 7) | L. MSH9 | >50 |
| | | 8) | L. oakridgenis (Oakridge 10) | >50 |
| | | 9) | L. pneumophila (PHA 1) | 100 |
| | | 10) | L. Lansing 2 | > 50 |
| | | 11) | L. SC-32C-C8 | >50 |
| III. | Other Bacterial Species | 1) | Aeormonas hydrophila | 1 |
| | | 2) | B. subtilis | 1 |
| | | 3) | Camplyobacter jejuni | 1 |
| | | 4) | Cytophaga johnsonae | 1 |
| | | 5) | E. coli | 1 |
| | | 6) | Flavobacterium breve | 1 |
| | | 7) | " gleum | 1 |
| | | 8) | " meningosepticum | 1 |
| | | 9) | " multivarum | 1 |
| | | 10) | Flavobacterium spiritovarum | 1 |
| | | 11) | " thalophohilum | 1 |
| | | 12) | Flexibacter canadensis | 1 |
| | | 13) | Proteus mirabilis | 1 |
| | | 14) | " rettgeri | 1 |
| | | 15) | " vulgaris | 1 |
| | | 16) | Providencia alicalifaciens | 1 |
| | | 17) | " stuartii | 1 |
| | | 18) | Pseudomonas alcaligenes | 1 |
| III. | Other Bacterial Species (cont'd) | 19) | Vibrio E1 Tor | 1 |
| | | 20) | Mycoplasma hominis | |
| | | 21) | " hyorhinis | 1 |
| | | 22) | " salivarium | 1 |
| | | 23) | Acholeplasma Laidlawii | 1 |
| | | 24) | Spiroplasma SMCA | 1 |
| | | 25) | " corn stunt | 1 |
| | | 26) | " honey bee | 1 |
| | | 27) | " cactus | 1 |
| IV. | Yeast | S. | cerevisiae | 1 |
| V. | Mammals | Human | | 1 |
| | | Hamster | | 1 |
| | | Mouse | | 1 |

Excess R-RNA hybridizations are done at 76° C, 0.48 M PB. Hybridization assays are done with hydroxyapatite at 72° C in 0.14 M PB, 0.005% sodium dodecyl sulfate. The hybridization exposure is sufficient to ensure complete reaction of the ³H-cDNA with nuclear R-RNA or for mitochondrial R-RNA. Non-bacterial R-RNA Cot's of at least 2 x 10³ are reached in the case of the mammals and birds.

**TABLE 7**

| Other Legionella Species Which Can Be Detected By Specific Nucleic Acid Probe of Table 7 |
|---|
| Species |
| L. WA-316 |
| L. WO-44-3C (L. feeleii) |
| L. Phoenix-1 |
| L. WA-270 A |
| L. PF-209C-C₂ |
| L. SC 65C3 (ORW) |
| L. Jamestown 26G1-E2 |
| L. MSH-4 |
| L. Lansing 3 |
| L. SC-18-C9 |
| L. SC-63-C7 |
| L. 81-716 (L. wadsworthii) |

### Example 3: Production of a Probe Which Will Hybridize Only to R-RNA from Members of the Genus Legionella

### Production of Radioactive DNA Complementary to Legionella R-RNA

R-RNA from the species Legionella pneumophila is used as a template to synthesize marked (radioactive) cDNA (complementary DNA) complementary to Legionella pneumophila R-RNA. This cDNA is produced by utilizing the ability of an enzyme, reverse transciptase, to utilize R-RNA as a template and produce ³H-cDNA complementary to R-RNA. This is done in the same way as described for producing M. hominis ³H-cDNA except that R-RNA from Legionella pneumophila is used as a template.

### Selection of Radioactive Probe which Hybridizes only to R-RNA from Members of the Genus Legionella

The purified ³H-cDNA is fractionated by hybridizing it with a great excess of R-RNA from E. coli, Acheolaplasma laidlawaii and Providentia stuartii. The hybridization mxiture consists of 0.05 - 1 micrograms of ³H-cDNA and 20 micrograms of each bacterial R-RNA in 1 ml of 0.48 M PB. This mixture is incubated at 76° C for 1 hour and the mixture is then diluted to 0.14 M PB and passed over HA equilibrated to 72° C, 0.14 M PB. The fraction of ³H-cDNA which does not adsorb to the HA (i.e., the ³H-cDNA not hybridized to the R-RNA) is collected. This fraction is then passed over HA under the same conditions as above and again the non-adsorbed fraction is collected. This ³H-cDNA is then concentrated and again hybridized with bacterial R-RNA as described above. The non-adsorbed fraction is collected and concentrated and then hybridized with bacterial R-RNA for a third time as described above and fractionated on HA as above. The non-adsorbed fraction is collected, base treated to remove any R-RNA present and concentrated into water. This ³H-cDNA preparation will hybridize to any member of the Legionella genus and will not hybridize to R-RNAs from other sources.

### Hybridization of Legionella specific ³H-cDNA Probe with R-RNA and R-RNA Genes from Different Sources

The selected probe allows the detection of any member of the genus Legionella in a sample by detecting the presence of Legionella R-RNA by nucleic acid hybridization. A necessary requirement of such a test is that the Legionella specific probe must not hybridize to R-RNA from other sources.

### Quantitation of Legionella R-RNA by Nucleic Acid Hybridization Excess R-RNA Method:

The amount of bacterial R-RNA present in a sample can be determined by measuring the kinetics of hybridization of the selected ³H-cDNA probe with the RNA isolated from a tissue sample and comparing these kinetics to those of a known standard mixture. This can be done even in the presence of a large excess of mammalian cell R-RNA since the probe does not hybridize with this R-RNA.

For measuring the kinetics, the hybridization mixture may contain, for example, 10⁻⁵ to 10⁻⁴ micrograms of ³H-cDNA and 0.01 to 10³ micrograms of purified sample RNA in 0.01 to 0.1 ml of 0.48 M PB. This mixture is incubated at 76° C and aliquots are removed, diluted to 0.14 M PB and assayed for hybridization at various times after the initiation of the reaction. Hybridization assays are performed using hydroxyapatite as described earlier. The results obtained are compared to the hybridization kinetics of the probe reacted with standard RNAs containing known amounts of bacterial R-RNA. These standards are mixtures of mammalian cell RNA and known amounts of a specific bacterial R-RNA.

Table 8 presents data on the quantitation of Legionella pneumophila present in water samples and in an infected hamster liver sample. The water samples and the liver samples were titered for the presence of L. pneumophila by standard quantitative'growth assays at the Center for Disease Control in Atlanta, Georgia.

**TABLE 8**

| | Measured by a Growth Assay | Measured by Excess R-RNA Nucleic Acid Hybridization |
|---|---|---|
| L. pneumophila bacteria per gram of infected hamster liver | 10⁷ bacteria gram liver | 1 - 2 x 10⁸ bacteria gram liver |
| L. pneumophila bacteria per ml of water sample | 1.5 x 10⁸ bacteria ml | 2.1 x 10⁸ bacteria ml |

### Excess Probe Method

The amount of bacterial R-RNA present in a sample can also be measured by doing hybridization under con-ditions where there is an excess of the Legionella specific ³H-cDNA probe, relative to the amount of Legionella R-RNA present. This mixture is hybridized to completion. At this point each Legionella R-RNA molecule present in the sample is saturated with probe molecules. By comparing the amount of probe hybridized to the R-RNA to an appropriately constructed standard calibration curve, the amount of R-RNA in a sample can be determined. A good estimate of the total number of Legionella pneumophila bacteria present in the sample can then be calculated by knowing the average number of R-RNA molecules per L. pneumophila bacterium.

Table 9 presents data on the quantitation of L. pneumophila present in water samples as determined by the excess probe accelerated hybridization rate - enzyme-detergent-sample method described in detail in a later. section. The water samples were titered for the presence of L. pneumophila by standard quantitative growth assays. These assays take days to complete while the hybridization assay takes about 1 hour.

**TABLE 9**

| | Measured by Growth Assay | Measured by the Excess Probe Method |
|---|---|---|
| L. pneumophila bacterial per ml water sample | 1.5 x 10⁸ bacteria ml | 2.2 x 10⁸ bacteria ml |

### Probe Specific Only for R-RNA from Members of the Genus Legionella

### A. Analysis of a Water Sample: Accelerated Hybridization Rate Method

1. Preparation of Sample and Hybridization Incubation Mixture:
   Mix in the following order as quickly as possible.
   a) 9 microliters of sample
   b) 2 microliters of enzyme-detergent solution containing: 5 milligrams/ml Proteinase K, 0.5 M Tris (pH = 8.1), 8% sodium dodecyl sulfate (SDS), 4% sodium sarcosinate, 0.25 M NaCl, 0.016 M EDTA, 0.016 EGTA
   c) 1 microliter of probe dissolved in water
   d) 20 microliters of 4.8 M PB
2. Incubate the mixture at 76° for an appropriate time so that the hybridization reaction is complete.
3. The hybridization assay is performed as follows:
   a) Add the incubation mixture to one ml of a room temperature solution containing: 0.05 grams hydroxyapatite (HA), 0.054 M PB, 0.02% Zwittergent 14 (CalBiochem) (hereinafter referred to as Z-14)
   b) Shake the mxiture for 30 seconds at room temperature, add 5 ml 0.14 M PB, 0.02% Z-14, and incubate the mixture at 72° C for 2 minutes.
   c) Centrifuge the solution to pellet the HA. All centrifugations are done at room temperature. Decant and save the liquid fraction. This is wash #1.
   d) Add 6 ml of 0.14 M PB, 0.02% Z-14 solution to the pellet. Resuspend the HA pellet by vortexing it. Centrifuge to pellet the HA and decant and save the liquid fraction. This is wash # 2.
   e) Repeat step d. This results in wash #3.
   f) Add 6 ml 0.03 and resuspend the HA pellet by vortexing. Centrifuge the suspension to pellet the HA and decant the liquid and assay it for the presence of the probe. This fraction contains the hybridized probe, if any is present.

It is not necessary to elute the hybridized probe from the HA under certain conditions. Thus, if the probe is marked with a marker which can be detected in the presence of HA, the. pellet from step e can be assayed directly for the probe. In the case of a marker such as Iodine-125 the tube containing the HA pellet can be placed directly into a gamma detection machine.

Other modifications can also be made to make the test faster and easier. Thus, 'the volume and amount of HA used can be scaled down, and the number of washes can also be reduced, depending on the situation. In other instances it may be desirable to increase the volumes of HA or number of washes. A variety of salts other than sodium phosphate, and other detergents can also be used in the assay.

### B. Analysis of a Liquid Sample: Standard Hybridization Rate Mathod

1. Preparation of Sample and Hybridization Inubation mix.
   Mix in the following order and as quickly as possible.
   a) 14 microliters of sample
   b) 2 microliters of enzyme-detergent solution described in A.
   c) L microliter of probe
   d) 3 microliters of 3.2 M PB, 0.03 M EDTA, 0.03 M EGTA
2. Incubate the mixture at 76° C for an appropriate time so that hybridization will complete.
3. The hybridization assay is performed as follows: .
   a) Add the incubation mix to 1 ml of a solution containing 0.14 M PB, 0.02% Z-14, 0.05 grams of HA.
   b) From this point on the protocol is identical to that described in A.

### C. Analysis of Tissue Sample: Accelerated Hybridization Rate Method

A 10 percent liver homogenate in water is used as the tissue sample,
1. Preparation of Sample and Incubation Mix.
   Mix as quickly as possible in the following order.
   a) 8 microliters of sample (10% liver homogenate)
   b) 3 milliliters of enzyme-detergent mix containing: 8% SDS, 4% sodium sarcosinate, 0,25 M NaCl, .016 M EDTA, 0,016 M EGTA, 0.38 M Tris (pH = 8.2), 195 milligrams/ml of Pronase.
   c) 1 microliter of probe specific only for Legionella R-RNA.
   d) 20 microliters of 4.8 M PB.
2. Incubate the mixture at 76° for an appropriate time to ensure that the hybridization reaction is complete.
3. The hybridization assay is performed as described in the section on analysis of a water sample; Accelerated Rate Method.

### D. Analysis. of Tissue Sample: Standard Hybridization Rate Method

A 10 percent liver homogenate in water used as the sample.
1. Preparation of the Sample and Incubation Mix.
   Mix as quickly as possible in the following order.
   a) 12 microliters of sample.
   b) 4 microliters of enzyme-detergent solution described in B.
   c) 1 microliter of probe specific only for Legionella R-RNA.
   d) 3 microliters of 3.2 M PB, 0.03 M EDTA, 0.03 M EGTA.
2. Incubate the mixe for an appropriate time at 76° C.
3. The hybridization assay is performed as follows.
   a) add the incubation mix to 1 ml of a solution containing 0.14 M PB, 0.02% Z-14, 0.05 grams HA.
   b) From this point the assay is identical to that described in A.

A more detailed description of nucleic acid hybridization tests to detect Legionella pneumophila bacteria in water and liver samples is presented below.

### Example 4

### Rapid Sensitive Detection of Legionella Bacteria in Water Sample: Accelerated Rate Method

1. The following components were mixed as quickly as possible and in the following order :
   a) 4.5 microliters of a water sample containing about 10⁵ Legionella pneumophila bacteria per ml. The number of bacteria in the water was determined at the Center of Disease Control in Atlanta by a growth test.
   b) 1 microliter of the enzyme-detergent solution described in A.
   c) 0.5 microliters of Legionella specific probe. The amount of probe equalled 7.5 x 10⁻⁶ micrograms.
   d) 10 microliters of 4.8 M PB.
   Assembling the hybridization mixture about 2 minutes.
2. Incubate the hyrbidization mixture for 36 minutes at 76° C.
3. Perform the hybridization assay as described in A. This took about 5 minutes.
4. Assay the fractions for the presence of the probe. This took about 10 minutes.

The number of Legionella bacteria present in the hybridization mixture was about 500. This number of organisms was detected and quantitated in about one hour, from start to finish, with the use of less than 10⁻⁵ micrograms of probe. Twenty-three percent of the probe hybridized with the Legionella R-RNA in this test which was designed to be an excess probe hybridization test. Control tests were done under the same conditions, one with no bacteria added, and one with about 10⁵ E. coli bacteria present in the hybridization mix. In both cases only 1-2 percent of the probe behaved as if it were hybridized.

The above test can be modified to assay larger volumes for the presence of Legionella bacteria. Thus, one ml of a water sample containing 10⁴ Legionella bacteria per ml was centrifuged for 30 minutes to pellet the bacteria. A small amount of enzyme-detergent was added to the pellet and a hybridization test was performed on this mixture using the accelerated rate method and the Legionella bacteria were readily detected. Much larger volumes of sample can be centrifuged and other methods of concentrating the bacteria, including membrane filtration, can also be used. These modifications make it possible to detect a small number of bacteria in a large sample volume. Air samples can also be concentrated by methods, including membrane filtration methods, and small numbers of bacteria can be detected in large volumes of air sample.

### Example 5

### Rapid Sensitive. Detection of Legionella Bacteria in a Hamster Liver Sample

1. The following components were mixed as quickly as possible in the following order:
   a) 4 microliters of a 10 percent liver homogenate of a hamster liver infected with Legionella pneumophila. This is equivalent to 400 micrograms of liver or about 6 x 10⁴ liver cells. About 750 Legionella pneumophila were present in this sample.
   b) 4 microliters of an enzyme-detergent solution composed of: 45 milligrams/ml Proteinase K, 8% SDS, 4% sodium sarcosinate, 0.5 M Tris (pH = 8.2), 0.008 M EDTA, 0.008 M EGTA, 0.25 M Nacl.
   c) 4 microliters of Legionella specific probe. The quantity of probe was about 10⁻⁵ micrograms.
2. Incubate the hybridization mixture at 76° C for 3 hours.
3. Perform the hybridization assay as described in A.
4. Assay the resulting fractions for the presence of probe hybridized to Legionella R-RNA.

The number of Legionella bacteria present in the hybridization mixture was about 750 and the amount of R-RNA present in this number of Legionella cells is about 1.1 x 10⁻⁵ micrograms. The number of liver cells present was about 6 x 10⁴ and the amount of liver R-RNA present was about one microgram. Ten percent of the Legionella specific probe hybridized. Control tests were done with uninfected liver in the same manner and less than one percent of the probe behaved as if hybridized. Examples 4 and 5 illustrate only two of the many possible configurations for such a test. Tests utilizing different volumes, salts, detergents, probes, sample types, proteolytic enzymes, amounts of HA, incubation periods, organism types, amounts of probe, temperatures of incubation, and hybridization rate accelerating systems can be successfully utilized within the general context of the tests described here. Any of the R-RNA probes can be used in a system comparable to those described above. Non R-RNA probes can also be used to good effect in these systems with some obvious modifications. For example, a test specific for a particular DNA sequence in a specific organism or group of organisms can be done exactly as described above if a step is included which converts the double strand DNA to the single strand form. In other cases different modifications of the method must be used. Bacteria such as Mycobacteria and Bacilli are difficult to break open. A step which breaks open these bacteria must then be used in conjunction with the method described above. A single incubation, in the absence of detergents, with the enzyme lysozyme, will make most Bacillus bacteria susceptible to lysis by detergents, for example. On the other hand, Mycobacteria are very difficult to lyse and may have to be physically broken open before they can be tested for.

A step designed to concentrate small numbers of bacteria or other cells out of large volumes of samples such as air or liquid can also be used in conjunction with the hybridization test to detect most other bacterial organisms or other types of organisms.

While I have described above, in detail, the production and use of a nucleic acid probe which hybridizes only to nucleic acids from members of the genus Legionella, it will be readily apparent to those skilled in the art from that example and the others, that other probes can be produced, based on the procedures illustrated above. Thus the method used to produce such other probes would be as follows:
1. Produce marked nucleic acid complementary to the R-RNA of a member of the group of interest.
2. Hybridize this DNA to the R-RNA from a member of the group of organisms evolutionarily most closely related to the group of organisms for which the probe is specific. Select the fraction of the marked nucleic acid which, at a specific criterion does not hybridize to R-RNA from a member of this closest related group of organisms. This fraction is specific for the organism group of interest.

### Examples of these are:

a. The production of a marked probe which hybridizes only with R-RNA from a member of the bacterial genus Leptospira and does not hybridize with R-RNA other sources.
b. The production of a marked probe which hybridizes only with R-RNA from a member of the bacterial genus Mycoplasma and does not hybridize with R-RNA from other sources.
c. The production of a marked probe which hybridizes only with R-RNA from a member of the bacterial family Enterobacteriaceae and does not hybridize with R-RNA from other sources.
d. The production of a marked probe which hybridizes only with R-RNA from a member of the anaerobic group of bacteria and does not hybridize with R-RNA from other sources.
e. The production of a marked probe which hybridizes only with R-RNA from a member of the group Fungi and does not hybridize with R-RNA from other sources.
f. The production of a marked probe which hybridizes only with R-RNA from any member of the Chlamydia group and does not hybridize with R-RNA from other sources.
g. The production of a'marked probe which hybridizes only with R-RNA from any member of the bacterial family Mycobacteriaceae and does not hybridize with R-RNA from other sources.
h. The production of a marked probe which hybridizes R-RNA from any living organism.
i. The production of marked probe which hybridizes only with R-RNA from any mammal and does not hybridize with R-RNA from other sources.

A useful initial screening test for tissue damage from any source can be done by utilizing a probe specific for R-RNA and examining blood or other body fluids for the presence of R-RNA sequences. Quantitation of R-RNA present will provide an indication as to the extent of tissue damage without identifying the source.

### The Determination of the Sensitivity of Microorganisms to Antimicroorganism Agents

A large number of different clinical situations require the determination of antimicrobial agent susceptibility for a variety of different bacteria and antibiotics (see "Antibiotics in Laboratory Medicine" by V. Lorian, Editor, Publisher, Williams, and Wilkens. Baltimore, 1980) All of these situations utilize a method for detecting and quantitating specific classes of microorganisms. In many of these situations use of the nucleic acid hybridization tests described earlier would greatly speed up the determination of antimicrobial agent susceptibility.

As the organisms in a sample grow and divide, the amount of RNA in the culture increases. A doubling of organisms results in a two fold increase in the quantity of RNA of different types which is present in the culture. Thus organism growth can be monitored by determining the quantity of RNA present in the culture at different times after the start of growth incubation. An increase in the amount of RNA present with time indicates organism growth. The magnitude of the increase indicates the extent of growth. The rate of growth is then the extent of growth per time period. Probes specific for R-RNA, can be used individually or in combination to measure the growth of organisms since the quantity of RNA in a culture will increase as the organisms grow.

A culture of specific category of organisms grown in the presence of an agent or agents which completely inhibit growth will not show an increase in RNA with time, while cultures which are partially inhibited by such agent will show a lower rate of RNA accumulation. A culture which is not inhibited will show the same rate of RNA increase as the control culture which does not contain the agent.

One example of this is in determining the susceptibility of Mycobacteria tubercules present in a clinical sputum sample. The first step in diagnosing such a sample is to prepare a direct smear of the sputum for staining in order to detect acid-fast bacilli. It is estimated that it requires at least 10⁴ - 10⁵ M. tuberculosis organisms per ml of sputum to yield a positive direct smear. However, only 10 to 100 of these organisms are recoverable by growth culture methods.

If the sputum specimen shows a positive smear, the specimen is then treated to kill all bacteria except Mycobacteria, and a dilution of the treated specimen is plated on agar medium containing antimicrobial agent and on control agar which does not contain the agent. Viable individual bacteria will form colonies on the control agar while growth will be inhibited on the agar with the appropriate antimicrobial agent. The ratio of the numbers on the control agar to those on the agent treated agar is then a measure of the effectiveness of the antimicrobial agent.

A small colony will- contain at least 10⁶ bacteria. This means that at least 20 divisions are needed to form a colony from one bacterium and each division will take at least 12 hours, for a total of 240 hours or 10 days, at a minimum. In most cases it takes 2 - 4 times this long (3 to 6 weeks) for colonies to appear.

A method described earlier for Legionella, would greatly decrease the time needed for determining antimicrobial agent susceptibility. A probe specific only for R-RNA from members of the genus Mycobacterium could be used in such a test. Such a probe would allow quantitation and a detection sensitivity equal to that described earlier for Legionella. A nucleic acid hybridization test using the accelerated hybridization rate conditions and the excess probe mode of hybridization would easily allow the detection of about 200 Mycobacteria cells. A step would be added to ensure the disruption of the Mycobacteria cells so that the R-RNA would be free to hybridize. Mycobacteria do not readily lyse in the presence of enzyme-detergent solutions.

As mentioned above, a minimum positive sputum specimen (as determined by acid staining) contains about 10⁴ to 10⁵ Mycobacteria cells per ml and these 10 to 10² cells can be detected as colony forming units. For drug susceptibility studies on agar, enough Mycobacteria are added to the control and experimental agar surfaces to ensure that 40 to 50 colonies will appear on the control agar where no antimicrobial agent is present. If such a practice is followed when using a nucleic acid hybridization assay this means that the culture is started with about 50 Mycobacteria and it will then take about 3 - 4 cell divisions or about 2 - 3 days in order to obtain a detectable level of cells. If any significant inhibition of growth by the agent has occurred the control will be positive and the culture containing agent will be negative. It is clear that the use of the highly sensitive nucleic acid hybridization method can greatly reduce the time needed to determine susceptibility by 5 to 10 fold.

The above is just one example of the uses of nucleic acid hybridization tests such as those described for Legionella for determing antimicrobial agent sensitivities. The sensitivity of any microorganism can be determined by utilizing a combination of the standard growth methodology and an assay for microorganims based on nucleic acid hybridization. In addition, in many cases the specificity and sensitivity of the nucleic acid hybridization tests for mrcroorganisms allow the determination of antibiotic sensitivity of specific organisms even in the presence of a large excess of other microorganisms or eukaryotic cells.

It is obvious that the same approach can be used to determine the presence of antimicroorganism activity in blood, urine, other body fluids and tissues and other samples. In this case my nucleic acid hybridization procedure can be used to monitor and quantitate the effect of the blood, urine, or other sample on the growth of a specific group of microorganisms which are put into contact with said blood, urine or other samples under conditions where growth occurs if antimicrobial activity is not present.

### Method for Detecting Microorganism Infections by Examining on Organism's Phagocytic Cells

The extremely high sensitivity and specificity of detection characterizing the nucleic acid hybridization tests specific for R-RNA which have been described above, permits a simple solution to the problem of obtaining an appropriate clinical specimen for microorganism diagnosis. A simple blood test sample which contains the white blood cell (hereinafter referred to as WBC) fraction will suffice in a large number of cases.

One manner of using this WBC approach is to first hybridize the WBC sample with a marked probe which will hybridize to R-RNA from any member of the group of all bacteria but does not hybridize to R-RNA from any other source. Such a probe serves as a general screening device for any bacteria. Samples which are positive for bacterial R-RNA are then assayed with a hierarchy of other probes in order to further identify the bacteria which is present. For example, a probe which hybridizes to R-RNA from any member of the family Enterobacter but not to R-RNA from any other source can be used to detect or rule out Enterobacter bacteria while a probe specific only for anaerobic R-RNA would be used to detect anaerobes.

The above illustration is just one of many possible ways of using the WBCs as the primary clinical sample for the quick diagnosis of microorganism infections by nucleic acid hybridization. For example, depending on the clinical symptoms of the patient different combinations of probes would be used in order to obtain a diagnosis.
1. Repeated Sequences in DNA R. J. Britten and D.E. Kohne, Science (1968) 161 p 529
2. Kinetics of Renaturation of DNA J. G. Wetmur and N. Davidson, J. Mol. Biol. (1968) 31 p. 349
3. Hydroxyapatite Techniques *for* Nucleic Acid Reassociation D.E. Kohne and R.J. Britten, in Procedures in Nucleic Acid Research (1971), eds Cantoni and Davies, Harper and Row Vol 2, p 500
4. Hybridization of Denatured RNA and Small Fragments Transferred to Nitrocellulose P.S. Thomas, Proc. Natl. Acad. Sci. USA (1980) 77 p 5201
5. DNA-DNA Hybridization on Nitrocellulose Filters: General Considerations and Non-Ideal Kinetics R. Flavell et al., Wur. J. Biochem. (1974) 47 p 535
6. Assay of DNA-RNA Hyrbids by S₁ Nuclease Digestion and Adsorption to DEAE-Cellulose Filters I. Maxwell et al., Nucleic Acids Research (1978) 5 p 2033
7. Molecular Cloning: A Laboratory Manual T. Maniatis et al., Cold Spring Harbor Publication (1982)
8. Efficient Transcription of RNA into DNA by Avian Sarcoma Virus Polymerase J. Taylor et al.. Biochemica et Biophys. Acta (1976) 442 p 324
9. Use of Specific Radioactive Probes to Study Transcription and Replication of the Influenza Virus Genome J. Taylor et al., J. Virology (1977) 21 #2, p 530
10. Virus Detection by Nucleic Acid Hybridization: Examination of Normal and ALS Tissue for the Presence of Poliovirus D. Kohne et al., Journal of General Virology (1981) 56 p 223-233
11. Leukemogensis by Bovine Leukemia Virus R. Kettmann et al., Proc. Natl. Acad. Sci. USA (1982) 79 #8 p 2465-2469
12. Prenatal Diagnosis of a Thalassemia: Clinical Application of Molecular Hybridization Y. Kan et al., New England Journal of Medicine (1976) 295 #21 p 1165-1167
.13. Gene Deletions in a Thalassemia Prove that the 5' Locus is Funtional L. Pressley et al., Proc. Natl. Acad, Sci. USA (1980) 77 #6 p 3586-3589
14. Use of Synthetic Oligonucleotides as Hybridization Probes. S.V. Suggs et al., Proc. Natl. Acad. Sci. USA (1981) 78 p 6613
15. .Identification of Enterotoxigenic E. coli by Colony,. Hybridization Using 3 Enterotoxin Gene Probes S.L. Mosely el atl., J. of Infect. Diseases (1982) 145 #6 p 863
16. DNA Reassociation in the Taxonomy of Enteric Bacteria D. Brenner, Int. J. Systematic Bacteriology (1973) 23 #4 p 298-307
17. Comparative study of Ribosomal RNA Cistrons in Enterobacteria and Mycobacteria R. Moore et al., J. Bacteriology (1967) 94 p 1066-1074
18. Ribosomal RNA Similarities in the Classification of Rhodococcus and Related Taxa M. Mordarski et al., J. General Microbiology (1980) 118 p. 313-319
19. Retention of Common Nucleotide Sequences in the Ribosomal RNA DNA of Eukaryotes and Some of their Physical Characteristics J. Sinclair et al., Biochemistry (1971) 10 p 2761
20. Homologies Among Ribosomal RNA and Messenger RNA Genes in Chloroplasts, Mitochondria and E. coli H. Bohnert et al., Molecular and General Genetics (1980) 179 p 539-545
21. Heterogeneity of the Conserved Ribosomal RNA Sequences of Bacillus subtilis R. Doe et al., J. Bacteriology (1966) 92 #1 p 88
22. Isolation and Characterization of Bacterial Ribosomal RNA Cistrons D. Kohne, Biophysical Journal (1968) 8 #10 p 1104-1118
23. Taxonomic Relations Between Archaebacteria Including 6 Novel Genera Examined by Cross Hybridization of DNAs and 16S R-RNAs J. Tu et al., J. Mol. Evol. (1982) 18 p 109
24. R-RNA Cistron Homologies Among Hypohomicrobium and Various Other Bacteria, R. Moore, Canadian J. Microbiology (1977) 23 p 478
25. Conservation of Transfer RNA and 5S RNA Cistrons in Enterobacteriaceae D.J. Brenner et al., J. Bacteriology Vol 129 #3 (Mar 1977) p 1435
26. Seqeunce Homology of Mitochondrial Leucul-tRNA Cistron in Different Organisms S. Jakovcic et al., Biochemistry Vol. 14 #10 (May 20, 1975), p. 2037
27. Synthetic Deoxyoligonucleotides as General Probes for Chloroplast t-RNA Genes J.A. Nickoloff and R.B. Hallick, Nucleci Acids Research, Vol. 10 #24 (1982) p 8191-8210
28. Antibiotics in Laboratory Medicine V. Lorian ed, Williams and Wilkens (Baltimore/London) 1980
29. Diagnostic Microbiology Finegold and Martin, Editors, C.V. Mosby Co. (St. Louis) 1982
30. Spotblot: A Hybridization Assay for Specific DNA Sequences in Multiple Samples M. Cunningham, Analytical Biochemistry Vol. 128 (1983) p. 415
31. (29) Analysis of Repeating DNA Sequences by Reassociation R. Britten et al., in: Methods in Enzumology XXIX, page 363, Eds. Grossman and Moldave, Academic Press, New York (1974)
32. Studies on Nucleic Acid Reassociation Kinetics: Retarded Rate of Hybridiation of RNA with Excess DNA G. Galau et al., Proc. Natl. Acad. Sci. USA Vol. 74 #6 (1974) p 2306
33. Acceleration of DNA Renaturation Rates J. Wetmur, Biopolymers Vol. 14 (1975) p 2517
34. Room Temperature Method for Increasing the Rate of DNA Reassociation by Many Thousandfold: The Phenol Emulsion Reassociation Technique D. Kohne et al., Biochemistry Vol. 16 #24 (1977) p 5349
35. Molecular Biology D. Freifelder, Science Books International (Boston) Van Nostrand Reinhold Co. (New York) 1983
36. Gene Expression 2 B. Lewin, J. Wiley & Sons, Wiley-Interscience Publication (1980) New York
37. Gene Expression 1 B. Lewin, J. Wiley & Sons, Wiley-Interscience Publication (1974) New York

As used in the specification and claims the following terms are definited as follows:

**DEFINITION OF TERMS**

| | |
|---|---|
| base (see nucleotide) base pair mismatches (see imperfectly complementary base sequence) | |
| base seqeunce, (nucleotide sequence or gene sequence or polynucleotide sequence or single strand nucleic acid sequence) | A DNA or RNA molecule consisting of multiple bases. |
| complementary base pairs | Certain of the bases have a chemical affinity for each other and pair together, or are complementary to one another. The complementary base pairs are A:T and G:C in DNA and A:U in RNA. |
| complementary strands or complementary base sequences | Perfectly complementary nucleic acid molecules are nucleic acid molecules in which each base in one molecule is paired with its complementary base in the other strand, to form a stable helical double strand molecule. The individual strands are termed complementary strands. |
| criterion | Most precisely defined as the difference between the temperature of melting of the double strand nucleic acid and the temperature at which hybridization is done. The melting temperature of a |
| | double strand nucleic acid is determined primarily by the salt concentration of the solution. The criterion determines the degree of complementarity needed for two single strands to form a stable double strand molecule. The criterion can be described as highly stringent, or not very stringent. A highly stringent criterion requires that two interacting complementary sequences be highly complementary in sequence in order to form a stable double strand molecule. A poorly stringent criterion is one which allows relatively dissimilar complimentary strands to interact and form a double strand molecule. High stringency allows the presence of only a small fraction of base pair mismatches in a double strand molecule. A poorly stringent criterion allows a much larger fraction of base pair mismatches in the hybridization product. |
| denatured or dissociated nucleic acid | The bond between the paired bases in a double strand nucleic acid molecule can be broken, resulting in two single strand molecules, which then diffuse away from each other. |
| double strand nucleic acid | As it is found in the cell, most DNA is in the double strand state. The DNA is made up of two DNA molecules or strands wound helically around each other. The bases face inward and each base is specifically bonded to a complementary base in the other strand. For example, an A in one strand is always paired with a T in the other strand, while |
| | a G in one strand is paired with a C in the other strand. In a bacterial cell the double strand molecule is about 5 x 10⁶ base pairs long. Each of the bases in one strand of this molecule is paired with its base complement in the other strand. The base sequences of the individual double strand molecules are termed complementary strands. |
| hybridization (see nucleic and hybridization) | |
| imperfectly complementary base sequences (base pair mismatches) | Stable double strand molecules can be formed between two strands where a fraction of the bases in the one strand are paired with a non-complementary base in the other strand. |
| marked probe or marked sequence | Single strand nucleic acid molecules which are used to detect the presence of other nucleic acids by the process of nucleic acid hybridization. The probe molecules are marked so that they can be specifically detected. This is done by incorporating a specific marker molecule into the nucleic acid or by attaching a specific marker to the nucleic acid. The most effective probes are marked, single strand sequences, which cannot self hybridize but can hybridize only if the nucleic acid to be detected is present. A large number of different markers are available. These include radio-active and fluorescent molecules. |
| nucleic acid hybridization or hybridization (reassociation, or renaturation) | The bond between the two strands of a double strand molecule can be broken and the two single strands can be completely separated from each other. Under the proper conditions the complementary single strands can collide, recognize each other and reform the double strand helical molecule. This process of formation of double strand molecules from complementary single strand molecules is called nucleic acid hybridization. |
| | Nucleic acid hybridization also occurs between partially complementary single strands of RNA and DNA. |
| nucleotide, nucleotide base or base | Most DNA consists of sequences of only four nitrogeneous bases: adenine (A), thymine (T), guanine (G), and cytosine (C). Together these bases form the genetic alphabet, and long ordered sequences of them contain, in coded form, much of the information present in genes. |
| | Most RNA also consists of sequences of only four bases. However, in RNA, thymine is replaced by uridine (U). |
| reassociation | (see nucleic acid hybridization) |
| renaturation | (see nucleic acid hybridization) |
| ribosomal RNA or R-RNA | The RNA which is present in ribosomes. Virtually all ribosomes contain 3 single strand RNA subunits: one large, one medium-sized, and one small. |
| ribosome | A cellular particle (containing RNA and protein) necessary for protein synthesis. All life forms except viruses contain ribosomes. |
| R-RNA DNA or R-RNA gene | The base sequence in the DNA which codes for ribosomal RNA. Each R-RNA subunit is coded for by a separate gene. |
| R-RNA probe | A marked nucleic acid sequence which is complementary to R-RNA and therefore will hybridize with R-RNA to form a stable double strand molecule. |
| mRNA | Each individual mRNA is a direct gene product containing the information necessary to specify a particular protein. The machinery of the cell translates the sequence of the mRNA into a specific protein. Many different mRNAs exist in each cell. |
| hnRNA | A complex class of RNA sequences present in the nucleus of eukaryotic cells which includes precursor mRNA molecules. Most hnRNA sequences never leave the nucleus. The function of most of these molecules in unknown. |
| snRNA | A class of relatively stable small nuclear RNA molecules which are present primarily in the nucleus of eukaryotic cells in large numbers. |
| precursor RNA | Many RNA molecules in both prokaryotes and eukaryotes are synthesized as part of a large RNA molecules which is then processed to yield mature RNA molecules of various types and other smaller sequences which are apparently discarded. |
| precursor specific RNA (ps RNA) | The RNA sequences present in precursor mRNA, t-RNA, R-RNA, snRNA, and hnRNA which are not present in the mature R-RNA, t-RNA, mRNA, snRNA, and hnRNA molecules. |
| thermal stability of double strand nucleic acid molecules | The thermal stability or melting temperature at which half of a population of double strand molecules has been converted to the single strand form. |
| restriction enzymes | Components of the restriction-modification cellular defense system against foreign nucleic acids. These enzymes cut unmodified (e.g., methylated) double-stranded DNA at specific sequences which exhibit twofold symmetry about a point. |
| transfer RNA (t-RNA) | During protein synthesis individual amino acids are aligned in the proper order by various specific adaptor molecules or t-RNA molecules. Each amino acid is ordered by a different t-RNA species. |

While the invention has been described and illustrated in detail, it will be apparent to those skilled in the art that various changes, equivalents and alternatives are contemplated as may come within the scope of the appended claims.

## Claims

1. A method for determining the presence of any member of a specific category of non-viral organisms which may be present in a sample, comprising the steps of:
a) contacting the sample with a probe comprising a nucleic acid molecule which hybridises to an rRNA sequence, or the gene encoding said rRNA sequence, belonging to said organisms under hybridisation conditions,
wherein the probe is complementary to only a fraction of said rRNA sequence, or the gene encoding said rRNA sequence, belonging to said organisms under said conditions, and
wherein the probe does not hybridise to any sequence in said sample belonging to a non-target organism under said conditions;
b) incubating said sample and said probe together under said conditions for a predetermined period of time; and
c) assaying said sample for hybridisation of said probe as an indication of the presence of any member of said specific category of organisms in said sample.

2. The method of claim 1, wherein said nucleic acid molecule hybridises to said rRNA sequence.

3. The method of claim 1, wherein said nucleic acid molecule hybridises to the gene encoding said rRNA sequence.

4. The method of any preceding claim, wherein said nucleic acid molecule is isolated by a hybridisation selection process.

5. The method of any one of claims 1 to 3, wherein said nucleic acid molecule is identified by comparing known nucleotide sequences.

6. The method of claim 4, wherein said nucleic acid molecule is sequenced and subsequently chemically synthesised.

7. The method of claim 5, wherein said nucleic acid molecule is chemically synthesised.

8. The method of any preceding claim, wherein said probe is marked.

9. The method of any preceding claim, wherein said specific category of organisms belongs to a taxonomic group.

10. The method of claim 9, wherein said taxonomic group consists of all prokaryotic organisms.

11. The method of claim 9, wherein said taxonomic group consists of all eukaryotic organisms.

12. The method of any preceding claim, wherein said sample contains a bodily fluid or tissue.

13. The method of claim 12 , wherein said sample contains blood, spinal fluid, urine or sputum.

14. A nucleic acid probe for determining the presence of any member of a specific category of non-viral organisms which may be present in a sample, said probe comprising:
a nucleic acid molecule which hybridises to an rRNA sequence, or the gene encoding said rRNA sequence, belonging to said organisms under hybridisation conditions,
wherein said probe is complementary to only a fraction of said rRNA sequence, or the gene encoding said rRNA sequence, belonging to said organisms under said conditions, and
wherein said probe does not hybridise to any sequence in said sample belonging to a non-target organism under said conditions.

15. A probe according to claim 14, wherein said nucleic acid molecule hybridises to said rRNA sequence.

16. A probe according to claim 14, wherein said nucleic acid molecule hybridises to the gene encoding said rRNA sequence.

17. A probe according to any one of claims 14 to 16, wherein said nucleic acid molecule is isolated by a hybridisation selection process.

18. A probe according to any one of claims 14 to 16, wherein said nucleic acid molecule is identified by comparing known nucleotide sequences.

19. A probe according to claim 17, wherein said nucleic acid molecule is sequenced and subsequently chemically synthesised.

20. A probe according to claim 18, wherein said nucleic acid molecule is chemically synthesised.

21. A probe according to any one of claims 14 to 20, wherein said nucleic acid molecule is marked.

22. A probe according to any one of claims 14 to 21, wherein said specific category of organisms belongs to a taxonomic group.

23. A probe according to claim 22, wherein said taxonomic group consists of all prokaryotic organisms.

24. A probe according to claim 22, wherein said taxonomic group consists of all eukaryotic organisms.

## Patentansprüche

1. Verfahren zum Feststellen des Vorhandenseins eines Mitglieds einer speziellen Kategorie nicht-viraler Organismen, welche in einer Probe vorhanden sein können, mit den Stufen, in denen man
a) die Probe mit einer ein Nukleinsäuremolekül umfassenden Sonde in Kontakt bringt, welche unter Hybridisierungsbedingungen an eine zu dem Organismus gehörende rRNA-Sequenz oder das die rRNA-Sequenz codierende Gen hybridisiert,
wobei die Sonde unter diesen Bedingungen nur zu einem Bruchteil der zu dem Organismus gehörenden rRNA-Sequenz oder dem die rRNA-Sequenz codierenden Gen komplementär ist und
wobei die Sonde unter diesen Bedingungen an keine zu einem Nicht-Ziel-Organismus gehörende Sequenz in der Probe hybridisiert,
b) die Probe und die Sonde zusammen unter diesen Bedingungen für einen bestimmten Zeitraum inkubiert, und
c) die Probe bezüglich Hybridisierung der Sonde als ein Anzeichen für das Vorhandensein eines Mitglieds der speziellen Kategorie von Organismen in der Probe untersucht.

2. Verfahren nach Anspruch 1, wobei das Nukleinsäuremolekül an die rRNA-Sequenz hybridisiert.

3. Verfahren nach Anspruch 1, wobei das Nukleinsäuremolekül an das die rRNA-Sequenz codierende Gen hybridisiert.

4. Verfahren nach einem der vorangegangenen Ansprüche, wobei das Nukleinsäuremolekül mittels eines Hybridisierungsselektionsverfahrens isoliert wird.

5. Verfahren nach einem der Ansprüche 1 bis 3, wobei das Nukleinsäuremolekül durch Vergleichen bekannter Nukleotidsequenzen identifiziert wird.

6. Verfahren nach Anspruch 4, wobei das Nukleinsäuremolekül sequenziert und anschließend chemisch synthetisiert wird.

7. Verfahren nach Anspruch 5, wobei das Nukleinsäuremolekül chemisch synthetisiert wird.

8. Verfahren nach einem der vorangegangenen Ansprüche, wobei die Sonde markiert wird.

9. Verfahren nach einem der vorangegangenen Ansprüche, wobei die spezielle Kategorie von Organismen zu einer taxonomischen Gruppe gehört.

10. Verfahren nach Anspruch 9, wobei die taxonomische Gruppe aus allen prokaryontischen Organismen besteht.

11. Verfahren nach Anspruch 9, wobei die taxonomische Gruppe aus allen eukaryontischen Organismen besteht.

12. Verfahren nach einem der vorangegangenen Ansprüche, wobei die Probe ein Körperfluid oder -gewebe enthält.

13. Verfahren hach Anspruch 12, wobei die Probe Blut, Spinalfluid, Urin oder Sputum enthält.

14. Nukleinsäuresonde zur Feststellung des Vorhandenseins eines Mitglieds einer speziellen Kategorie nicht-viraler Organismen, welche in einer Probe vorhanden sein können, wobei die Sonde
ein Nukleinsäuremolekül enthält, welches unter Hybridisierungsbedingungen an eine zu dem Organismus gehörende rRNA-Sequenz oder das diese rRNA-Sequenz codierende Gen hybridisiert,
wobei die Sonde unter diesen Bedingungen nur zu einem Bruchteil der zu dem Organismus gehörenden rRNA-Sequenz oder dem diese rRNA-Sequenz codierenden Gen komplementär ist und
wobei die Sonde unter diesen Bedingungen an keine Sequenz in der Probe hybridisiert, die zu einem Nicht-Ziel-Organismus gehört.

15. Sonde nach Anspruch 14, wobei das Nukleinsäuremolekül an die rRNA-Sequenz hybridisiert.

16. Sonde nach Anspruch 14, wobei das Nukleinsäuremolekül an das die rRNA-Sequenz codierende Gen hybridisiert.

17. Sonde nach einem der Ansprüche 14 bis 16, wobei das Nukleinsäuremolekül mittels eines Hybridisierungsselektionsverfahrens isoliert wird.

18. Sonde nach einem der Ansprüche 14 bis 16, wobei das Nukleinsäuremolekül durch Vergleichen bekannter Nukleotidsequenzen identifiziert wird.

19. Sonde nach Anspruch 17, wobei das Nukleinsäuremolekül sequenziert und anschließend chemisch synthetisiert wird.

20. Sonde nach Anspruch 18, wobei das Nukleinsäuremolekül chemisch synthetisiert wird.

21. Sonde nach einem der Ansprüche 14 bis 20, wobei das Nukleinsäuremolekül markiert wird.

22. Sonde nach einem der Ansprüche 14 bis 21, wobei die spezielle Kategorie von Organismen zu einer taxonomischen Gruppe gehört.

23. Sonde nach Anspruch 22, wobei die taxonomische Gruppe aus allen prokaryontischen Organismen besteht.

24. Sonde nach Anspruch 22, wobei die taxonomische Gruppe aus allen eukaryontischen Organismen besteht.

## Revendications

1. Méthode pour déterminer la présence de n'importe quel membre d'une catégorie spécifique d'organismes non viraux qui peuvent être présents dans un échantillon, comprenant les étapes consistant :
a) à mettre en contact l'échantillon avec une sonde comprenant une molécule d'acide nucléique qui s'hybride à une séquence d'ARN-R, ou au gène codant pour ladite séquence d'ARN-R appartenant auxdits organismes dans des conditions d'hybridation,
la sonde étant complémentaire de seulement une fraction de ladite séquence d'ARN-R, ou du gène codant pour ladite séquence d'ARN-R, appartenant auxdits organismes dans lesdites conditions, et
la sonde ne s'hybridant pas à une quelconque séquence dans ledit échantillon appartenant à un organisme non cible dans lesdites conditions ;
b) à mettre en incubation ledit échantillon et ladite sonde ensemble dans lesdites conditions pendant une période de temps prédéterminée ; et
c) à analyser ledit échantillon pour déterminer l'hybridation de ladite sonde à titre d'indication de la présence d'un quelconque membre de ladite catégorie spécifique d'organismes dans ledit échantillon.

2. Méthode suivant la revendication 1, dans laquelle la molécule d'acide nucléique s'hybride à la séquence d'ARN-R.

3. Méthode suivant la revendication 1, dans laquelle la molécule d'acide nucléique s'hybride au gène codant pour la séquence d'ARN-R.

4. Méthode suivant l'une quelconque des revendications précédentes, dans laquelle la molécule d'acide nucléique est isolée par un procédé de sélection à des fins d'hybridation.

5. Méthode suivant l'une quelconque des revendications 1 à 3, dans laquelle la molécule d'acide nucléique est identifiée en comparant des séquences de nucléotides connues.

6. Méthode suivant la revendication 4, dans laquelle la molécule d'acide nucléique est séquencée et ensuite synthétisée chimiquement.

7. Méthode suivant la revendication 5, dans laquelle la molécule d'acide nucléique est synthétisée chimiquement.

8. Méthode suivant l'une quelconque des revendications précédentes, dans laquelle la sonde est marquée.

9. Méthode suivant l'une quelconque des revendications précédentes, dans laquelle la catégorie spécifique d'organismes appartient à un groupe taxonomique.

10. Méthode suivant la revendication 9, dans laquelle le groupe taxonomique consiste en l'ensemble des organismes procaryotiques.

11. Méthode suivant la revendication 9, dans laquelle le groupe taxonomique consiste en l'ensemble des organismes eucaryotiques.

12. Méthode suivant l'une quelconque des revendications précédentes, dans laquelle l'échantillon contient un liquide biologique ou tissu.

13. Méthode suivant la revendication 12, dans laquelle l'échantillon contient du sang, du liquide rachidien, de l'urine ou des expectorations.

14. Sonde d'acide nucléique pour déterminer la présence de n'importe quel membre d'une catégorie spécifique d'organismes non viraux qui peuvent être présents dans un échantillon, ladite sonde comprenant :
une molécule d'acide nucléique qui s'hybride à une séquence d'ARN-R, ou au gène codant pour ladite séquence d'ARN-R, appartenant auxdits organismes dans des conditions d'hybridation,
ladite sonde étant complémentaire de seulement une fraction de ladite séquence d'ARN-R, ou du gène codant pour ladite séquence d'ARN-R, appartenant auxdits organismes dans lesdites conditions, et
ladite sonde ne s'hybridant pas à une quelconque séquence dans ledit échantillon appartenant à un organisme non cible dans lesdites conditions.

15. Sonde suivant la revendication 14, dans laquelle la molécule d'acide nucléique s'hybride à la séquence d'ARN-R.

16. Sonde suivant la revendication 14, dans laquelle la molécule d'acide nucléique s'hybride au gène codant pour la séquence d'ARN-R.

17. Sonde suivant l'une quelconque des revendications 14 à 16, dans laquelle la molécule d'acide nucléique est isolée par un procédé de sélection à des fins d'hybridation.

18. Sonde suivant l'une quelconque des revendications 14 à 16, dans laquelle la molécule d'acide nucléique est identifiée en comparant des séquences de nucléotides connues.

19. Sonde suivant la revendication 17, dans laquelle la molécule d'acide nucléique est séquencée et ensuite synthétisée chimiquement.

20. Sonde suivant la revendication 18, dans laquelle la molécule d'acide nucléique est synthétisée chimiquement.

21. Sonde suivant l'une quelconque des revendications 14 à 20, dans laquelle la molécule d'acide nucléique est marquée.

22. Sonde suivant l'une quelconque des revendications 14 à 21, dans laquelle la catégorie spécifique d'organismes appartient à un groupe taxonomique.

23. Sonde suivant la revendication 22, dans laquelle le groupe taxonomique consiste en l'ensemble des organismes procaryotiques.

24. Sonde suivant la revendication 22, dans laquelle le groupe taxonomique consiste en l'ensemble des organismes eucaryotiques.
